# EUROPEAN PATENT SPECIFICATION

(11) **EP 0 907 730 B1**
(45) Date of publication and mention of the grant of the patent: **07.10.2009**
(21) Application number: 96934193.2
(22) Date of filing: 23.10.1996
(51) Int. Cl.: C12N 15/12, C12N 5/06, C07K 14/715, C07K 16/28, C07K 19/00, A61K 38/16, A61K 48/00, G01N 33/566, G01N 33/53

(54) **HAEMOPOIETIN RECEPTOR AND GENETIC SEQUENCES ENCODING SAME**
HEMOPOIETIN-REZEPTOR UND DAFÜR KODIERENDE GENETISCHE SEQUENZEN
RECEPTEUR D'HEMATOPOIETINE ET SEQUENCES GENETIQUES LE CODANT

(30) Priority: 23.10.1995 AU PN613595; 22.12.1995 AU PN727695; 09.09.1996 AU PO220896
(43) Date of publication of application: 14.04.1999
(73) Proprietor: Zenyth Operations Pty Ltd, Parkville VIC 3052 (AU)
(72) Inventor: WILLSON, Tracy, North Balwyn, VIC 3104 (AU); NICOLA, Nicos, A., Mont Albert, VIC 3127 (AU); HILTON, Douglas, J., Warrandyte, VIC 3113 (AU); METCALF, Donald, Balwyn, VIC 3103 (AU); ZHANG, Jian', Guo, Hoppers Crossing, VIC 3029 (AU)
(74) Representative: Jones, Elizabeth Louise
(86) International application number: PCT/AU1996/000668
(87) International publication number: WO 1997/015663

(56) References cited:
- WO-A-96/07737
- WO-A-96/11213
- WO-A-97/20926
- ZURAWSKI S M ET AL: "THE PRIMARY BINDING SUBUNIT OF THE HUMAN INTERLEUKIN-4 RECEPTOR IS ALSO A COMPONENT OF THE INTERLEUKIN-13 RECEPTOR" JOURNAL OF BIOLOGICAL CHEMISTRY,AMERICAN SOCIETY OF BIOLOGICAL CHEMISTS, BALTIMORE, MD,US, vol. 270, no. 23, 9 June 1995 (1995-06-09), pages 13869-13878, XP002010894 ISSN: 0021-9258
- HILTON D J ET AL: "CLONING OF A MURINE IL-11 RECEPTOR ALPHA-CHAIN;REQUIREMENT FOR GP130 FOR HIGH AFFINITY BINDING AND SIGNAL TRANSDUCTION" EMBO JOURNAL,GB,OXFORD UNIVERSITY PRESS, SURREY, vol. 13, no. 20, 17 October 1994 (1994-10-17), pages 4765-4775, XP000673205 ISSN: 0261-4189
- HILTON D J ET AL: "CLONING AND CHARACTERIZATION OF A BINDING SUBUNIT OF THE INTERLEUKIN 13 RECEPTOR THAT IS ALSO A COMPONENT OF THE INTERLEUKIN4 RECEPTOR" PROCEEDINGS OF THE NATIONAL ACADEMY OF SCIENCES OF USA,US,NATIONAL ACADEMY OF SCIENCE. WASHINGTON, vol. 93, no. 1, 9 January 1996 (1996-01-09), pages 497-501, XP002010896 ISSN: 0027-8424
- JAVAD AMAN M ET AL: "CDNA CLONING AND CHARACTERIZATION OF THE HUMAN INTERLEUKIN 13 RECEPTOR ALPHA CHAIN" JOURNAL OF BIOLOGICAL CHEMISTRY,US,AMERICAN SOCIETY OF BIOLOGICAL CHEMISTS, BALTIMORE, MD, vol. 271, no. 46, 15 November 1996 (1996-11-15), pages 29265-29270, XP002025818 ISSN: 0021-9258
- GUIDEBOOK TO CYTOKINES AND THEIR RECEPTORS, Edited by NICOLA N.A., 1994, OXFORD UNIVERSITY PRESS, OXFORD, pages 4,5,8,9,31-35,40-43,47-49,52-55,59-61,64-66 ,82-83,130-133,137-139,155-157,161-163,174- 176,191-193, XP002957020
- J. BIOL. CHEM., Vol. 270(8), 1995, VITA N. et al., "Characterization and Comparison of the Interleukin 13 Receptor With the Interleukin 4 Receptor on Several Cell Types", pages 3512-3517, XP002010892.
- PROC. NATL. ACAD. SCI USA., Vol. 87, 1990, HARADA N. et al., "Expression Cloning of a cDNA Encoding the Murine Interleukin 4 Receptor Based on Ligand Binding", pages 857-861, XP000095666.
- J. BIOL. CHEM., Vol. 271(28), CAPUT D. et al., "Cloning and Characterisation of a Specific Interleukin (IL) -13 Binding Protein Structurally Related to the IL-5 Receptor Alpha Chain", pages 16921-16926, XP002195292.
- KUWAKI T. ET AL: 'CHARACTERIZATION OF HUMAN INTERLEUKIN-3 RECEPTORS ON A MULTI-FACTOR-DEPENDENT CELL LINE' BIOCHEMICAL AND BIOPHYSICAL RESEARCH COMMUNICATIONS vol. 161, no. 1, 30 May 1989, pages 16 - 22, XP000051605
- KITAMURA T. ET AL: 'EXPRESSION CLONING OF THE HUMAN IL-3 RECEPTOR COMPLEMENTARY DNA REVEALS A SHARED BETA SUBUNIT FOR THE HUMAN IL-3 AND GM-CSF RECEPTORS' CELL vol. 66, no. 6, 20 September 1991, pages 1165 - 1174

### SEQUENCE LISTING

<110> AMRAD Operations Pty Ltd (all States except US)
   Willson (US only), Tracey
   Nicola (US only), Nicos
   Hilton (US only), Douglas
   Metcalf (US only), Donald
   Zhang (US only), Jian
<120> A novel haemopoietin receptor and genetic sequences encoding same
<130> 42.68173.ej
<140> EP 96934193.2
   <141> 1996-10-23
<150> AU PN6135
   <151> 1995-10-23
<150> AU PN7276
   <151> 1995-12-22
<150> AU PP2208
   <151> 1996-09-09
<160> 12
<170> PatentIn version 3.1
<210> 1
   <211> 1680
   <212> DNA
   <213> Mus musculus
<220>
   <221> CDS
   <222> (61)..(1332)
   <223>
<400> 1
   tgaaaagata gaataaatgg cctcgtgccg aattcggcac gagccgaggc gagggcctgc      60
   atg gcg cgg cca gcg ctg ctg ggc gag ctg ttg gtg ctg cta ctg tgg       108
   Met Ala Arg Pro Ala Leu Leu Gly Glu Leu Leu Val Leu Leu Leu Trp
   1               5                   10                  15
   acc gcc acc gtg ggc caa gtt gcc gcg gcc aca gaa gtt cag cca cct       156
   Thr Ala Thr Val Gly Gln Val Ala Ala Ala Thr Glu Val Gln Pro Pro
               20                  25                  30
   gtg acg aat ttg agc gtc tct gtc gaa aat ctc tgc acg ata ata tgg       204
   Val Thr Asn Leu Ser Val Ser Val Glu Asn Leu Cys Thr Ile Ile Trp
           35                  40                  45
   acg tgg agt cct cct gaa gga gcc agt cca aat tgc act ctc aga tat       252
   Thr Trp Ser Pro Pro Glu Gly Ala Ser Pro Asn Cys Thr Leu Arg Tyr
       50                  55                  60
   ttt agt cac ttt gat gac caa cag gat aag aaa att gct cca gaa act      300
   Phe Ser His Phe Asp Asp Gln Gln Asp Lys Lys Ile Ala Pro Glu Thr
   65                  70                  75                  80
   cat cgt aaa gag gaa tta ccc ctg gat gag aaa atc tgt ctg cag gtg      348
   His Arg Lys Glu Glu Leu Pro Leu Asp Glu Lys Ile Cys Leu Gln Val
                   85                  90                  95
   ggc tct cag tgt agt gcc aat gaa agt gag aag cct agc cct ttg gtg      396
   Gly Ser Gln Cys Ser Ala Asn Glu Ser Glu Lys Pro Ser Pro Leu Val
               100                 105                 110
   aaa aag tgc atc tca ccc cct gaa ggt gat cct gag tcc gct gtg act      444
   Lys Lys Cys Ile Ser Pro Pro Glu Gly Asp Pro Glu Ser Ala Val Thr
           115                 120                 125
   gag ctc aag tgc att tgg cat aac ctg agc tat atg aag tgt tcc tgg      492
   Glu Leu Lys Cys Ile Trp His Asn Leu Ser Tyr Met Lys Cys Ser Trp
       130                 135                 140
   ctc cct gga agg aat aca agc cct gac aca cac tat act ctg tac tat      540
   Leu Pro Gly Arg Asn Thr Ser Pro Asp Thr His Tyr Thr Leu Tyr Tyr
   145                 150                 155                 160
   tgg tac agc agc ctg gag aaa agt cgt caa tgt gaa aac atc tat aga      588
   Trp Tyr Ser Ser Leu Glu Lys Ser Arg Gln Cys Glu Asn Ile Tyr Arg
                   165                 170                 175
   gaa ggt caa cac att gct tgt tcc ttt aaa ttg act aaa gtg gaa cct      636
   Glu Gly Gln His Ile Ala Cys Ser Phe Lys Leu Thr Lys Val Glu Pro
               180                 185                 190
   agt ttt gaa cat cag aac gtt caa ata atg gtc aag gat aat gct ggg      684
   Ser Phe Glu His Gln Asn Val Gln Ile Met Val Lys Asp Asn Ala Gly
           195                 200                 205
   aaa att agg cca tcc tgc aaa ata gtg tct tta act tcc tat gtg aaa      732
   Lys Ile Arg Pro Ser Cys Lys Ile Val Ser Leu Thr Ser Tyr Val Lys
       210                 215                 220
   cct gat cct cca cat att aaa cat ctt ctc ctc aaa aat ggt gcc tta      780
   Pro Asp Pro Pro His Ile Lys His Leu Leu Leu Lys Asn Gly Ala Leu
   225                 230                 235                 240
   tta gtg cag tgg aag aat cca caa aat ttt aga agc aga tgc tta act      828
   Leu Val Gln Trp Lys Asn Pro Gln Asn Phe Arg Ser Arg Cys Leu Thr
                   245                 250                 255
   tat gaa gtg gag gtc aat aat act caa acc gac cga cat aat att tta      876
   Tyr Glu Val Glu Val Asn Asn Thr Gln Thr Asp Arg His Asn Ile Leu
               260                 265                 270
   gag gtt gaa gag gac aaa tgc cag aat tcc gaa tct gat aga aac atg      924
   Glu Val Glu Glu Asp Lys Cys Gln Asn Ser Glu Ser Asp Arg Asn Met
           275                 280                 285
   gag ggt aca agt tgt ttc caa ctc cct ggt gtt ctt gcc gac gct gtc     972
   Glu Gly Thr Ser Cys Phe Gln Leu Pro Gly Val Leu Ala Asp Ala Val
       290                 295                 300
   tac aca gtc aga gta aga gtc aaa aca aac aag tta tgc ttt gat gac    1020
   Tyr Thr Val Arg Val Arg Val Lys Thr Asn Lys Leu Cys Phe Asp Asp
   305                 310                 315                 320
   aac aaa ctg tgg agt gat tgg agt gaa gca cag agt ata ggt aag gag    1068
   Asn Lys Leu Trp Ser Asp Trp Ser Glu Ala Gln Ser Ile Gly Lys Glu
                   325                 330                 335
   caa aac tcc acc ttc tac acc acc atg tta ctc acc att cca gtc ttt    1116
   Gln Asn Ser Thr Phe Tyr Thr Thr Met Leu Leu Thr Ile Pro Val Phe
               340                 345                 350
   gtc gca gtg gca gtc ata atc ctc ctt ttt tac ctg aaa agg ctt aag    1164
   Val Ala Val Ala Val Ile Ile Leu Leu Phe Tyr Leu Lys Arg Leu Lys
           355                 360                 365
   atc att ata ttt cct cca att cct gat cct ggc aag att ttt aaa gaa    1212
   Ile Ile Ile Phe Pro Pro Ile Pro Asp Pro Gly Lys Ile Phe Lys Glu
       370                 375                 380
   atg ttt gga gac cag aat gat gat acc ctg cac tgg aag aag tat gac    1260
   Met Phe Gly Asp Gln Asn Asp Asp Thr Leu His Trp Lys Lys Tyr Asp
   385                 390                 395                 400
   atc tat gag aaa caa tcc aaa gaa gaa acg gat tct gta gtg ctg ata    1308
   Ile Tyr Glu Lys Gln Ser Lys Glu Glu Thr Asp Ser Val Val Leu Ile
                   405                 410                 415
   gaa aac ctg aag aaa gca gct cct tgatggggag aagtgatttc tttcttgcct   1362
   Glu Asn Leu Lys Lys Ala Ala Pro
               420
   tcaatgtgac cctgtgaaga tttattgcat tctccatttg ttatctgggg gacttgttaa  1422
   atagaaactg aaactactct tgaaaaacag gcagctccta agagccacag gtcttgatgt  1482
   gacttttgca ttgaaaaccc aaacccaaag gagctccttc caagaaaagc aagagttctt  1542
   ctcgttcctt gttccaatcc ctaaaagcag atgttttgcc aaatccccaa actagaggac  1602
   aaagacaagg ggacaatgac catcaattca tctaatcagg aattgtgatg gcttcctaag  1662
   gaatctctgc ttgctctg                                                1680
<210> 2
   <211> 424
   <212> PRT
   <213> Mus musculus
<400> 2
   Met Ala Arg Pro Ala Leu Leu Gly Glu Leu Leu Val Leu Leu Leu Trp
   1 5 10 15
   Thr Ala Thr Val Gly Gln Val Ala Ala Ala Thr Glu Val Gln Pro Pro
   20 25 30
   Val Thr Asn Leu Ser Val Ser Val Glu Asn Leu Cys Thr Ile Ile Trp
   35 40 45
   Thr Trp Ser Pro Pro Glu Gly Ala Ser Pro Asn Cys Thr Leu Arg Tyr
   50 55 60
   Phe Ser His Phe Asp Asp Gln Gln Asp Lys Lys Ile Ala Pro Glu Thr
   65 70 75 80
   His Arg Lys Glu Glu Leu Pro Leu Asp Glu Lys Ile Cys Leu Gln Val
   85 90 95
   Gly Ser Gln Cys Ser Ala Asn Glu Ser Glu Lys Pro Ser Pro Leu Val
   100 105 110
   Lys Lys Cys Ile Ser Pro Pro Glu Gly Asp Pro Glu Ser Ala Val Thr
   115 120 125
   Glu Leu Lys Cys Ile Trp His Asn Leu Ser Tyr Met Lys Cys Ser Trp
   130 135 140
   Leu Pro Gly Arg Asn Thr Ser Pro Asp Thr His Tyr Thr Leu Tyr Tyr
   145 150 155 160
   Trp Tyr Ser Ser Leu Glu Lys Ser Arg Gln Cys Glu Asn Ile Tyr Arg
   165 170 175
   Glu Gly Gln His Ile Ala Cys Ser Phe Lys Leu Thr Lys Val Glu Pro
   180 185 190
   Ser Phe Glu His Gln Asn Val Gln Ile Met Val Lys Asp Asn Ala Gly
   195 200 205
   Lys Ile Arg Pro Ser Cys Lys Ile Val Ser Leu Thr Ser Tyr Val Lys
   210 215 220
   Pro Asp Pro Pro His Ile Lys His Leu Leu Leu Lys Asn Gly Ala Leu
   225 230 235 240
   Leu Val Gln Trp Lys Asn Pro Gln Asn Phe Arg Ser Arg Cys Leu Thr
                   245                 250                 255
   Tyr Glu Val Glu Val Asn Asn Thr Gln Thr Asp Arg His Asn Ile Leu
               260                 265                 270
   Glu Val Glu Glu Asp Lys Cys Gln Asn Ser Glu Ser Asp Arg Asn Met
           275                 280                 285
   Glu Gly Thr Ser Cys Phe Gln Leu Pro Gly Val Leu Ala Asp Ala Val
       290                 295                 300
   Tyr Thr Val Arg Val Arg Val Lys Thr Asn Lys Leu Cys Phe Asp Asp
   305                 310                 315                 320
   Asn Lys Leu Trp Ser Asp Trp Ser Glu Ala Gln Ser Ile Gly Lys Glu
                   325                 330                 335
   Gln Asn Ser Thr Phe Tyr Thr Thr Met Leu Leu Thr Ile Pro Val Phe
               340                 345                 350
   Val Ala Val Ala Val Ile Ile Leu Leu Phe Tyr Leu Lys Arg Leu Lys
           355                 360                 365
   Ile Ile Ile Phe Pro Pro Ile Pro Asp Pro Gly Lys Ile Phe Lys Glu
       370                 375                 380
   Met Phe Gly Asp Gln Asn Asp Asp Thr Leu His Trp Lys Lys Tyr Asp
   385                 390                 395                 400
   Ile Tyr Glu Lys Gln Ser Lys Glu Glu Thr Asp Ser Val Val Leu Ile
                   405                 410                 415
   Glu Asn Leu Lys Lys Ala Ala Pro
               420
<210> 3
   <211> 1383
   <212> DNA
   <213> human
<220>
   <221> CDS
   <222> (61)..(1338)
   <223>
<400> 3
   gagtctaaca cggaccaagg agtttaacac gtgcggccgg gttccgaggc gagaggctgc       60
   atg gag tgg ccg gcg cgg ctc tgc ggg ctg tgg gcg ctg ctg ctc tgc        108
   Met Glu Trp Pro Ala Arg Leu Cys Gly Leu Trp Ala Leu Leu Leu Cys
   1               5                   10                  15
   gcc ggc ggc ggg ggc ggg ggc ggg ggc gcg cct acg gaa act cag cca        156
   Ala Gly Gly Gly Gly Gly Gly Gly Gly Ala Pro Thr Glu Thr Gln Pro
               20                  25                  30
   cct gtg aca aat ttg agt gtc tct gtt gaa aac ctc tgc aca gta ata        204
   Pro Val Thr Asn Leu Ser Val Ser Val Glu Asn Leu Cys Thr Val Ile
           35                  40                  45
   tgg aca tgg aat cca ccc gag gga gcc agc tca aat tgt agt cta tgg        252
   Trp Thr Trp Asn Pro Pro Glu Gly Ala Ser Ser Asn Cys Ser Leu Trp
       50                  55                  60
   tat ttt agt cat ttt ggc gac aaa caa gat aag aaa ata gct ccg gaa        300
   Tyr Phe Ser His Phe Gly Asp Lys Gln Asp Lys Lys Ile Ala Pro Glu
   65                  70                  75                  80
   act cgt cgt tca ata gaa gta ccc ctg aat gag agg att tgt ctg caa        348
   Thr Arg Arg Ser Ile Glu Val Pro Leu Asn Glu Arg Ile Cys Leu Gln
                   85                  90                  95
   gtg ggg tcc cag tgt agc acc aat gag agt gag aag cct agc att ttg        396
   Val Gly Ser Gln Cys Ser Thr Asn Glu Ser Glu Lys Pro Ser Ile Leu
               100                 105                 110
   gtt gaa aaa tgc atc tca ccc cca gaa ggt gat cct gag tct gct gtg        444
   Val Glu Lys Cys Ile Ser Pro Pro Glu Gly Asp Pro Glu Ser Ala Val
           115                 120                 125
   act gaa ctt caa tgc att tgg cac aac ctg agc tac atg aag tgt tct        492
   Thr Glu Leu Gln Cys Ile Trp His Asn Leu Ser Tyr Met Lys Cys Ser
       130                 135                 140
   tgg ctc cct gga agg aat acc agt ccc gac act aac tat act ctc tac        540
   Trp Leu Pro Gly Arg Asn Thr Ser Pro Asp Thr Asn Tyr Thr Leu Tyr
   145                 150                 155                 160
   tat tgg cac aga agc ctg gaa aaa att cat caa tgt gaa aac atc ttt        588
   Tyr Trp His Arg Ser Leu Glu Lys Ile His Gln Cys Glu Asn Ile Phe
                   165                 170                 175
   aga gaa ggc caa tac ttt ggt tgt tcc ttt gat ctg acc aaa gtg aag        636
   Arg Glu Gly Gln Tyr Phe Gly Cys Ser Phe Asp Leu Thr Lys Val Lys
               180                 185                 190
   gat tcc agt ttt gaa caa cac agt gtc caa ata atg gtc aag gat aat        684
   Asp Ser Ser Phe Glu Gln His Ser Val Gln Ile Met Val Lys Asp Asn
           195                 200                 205
   gca gga aaa att aaa cca tcc ttc aat ata gtg cct tta act tcc cgt      732
   Ala Gly Lys Ile Lys Pro Ser Phe Asn Ile Val Pro Leu Thr Ser Arg
       210                 215                 220
   gtg aaa cct gat cct cca cat att aaa aac ctc tcc ttc cac aat gat      780
   Val Lys Pro Asp Pro Pro His Ile Lys Asn Leu Ser Phe His Asn Asp
   225                 230                 235                 240
   gac cta tat gtg caa tgg gag aat cca cag aat ttt att agc aga tgc      828
   Asp Leu Tyr Val Gln Trp Glu Asn Pro Gln Asn Phe Ile Ser Arg Cys
                   245                 250                 255
   cta ttt tat gaa gta gaa gtc aat aac agc caa act gag aca cat aat      876
   Leu Phe Tyr Glu Val Glu Val Asn Asn Ser Gln Thr Glu Thr His Asn
               260                 265                 270
   gtt ttc tac gtc caa gag gct aaa tgt gag aat cca gaa ttt gag aga      924
   Val Phe Tyr Val Gln Glu Ala Lys Cys Glu Asn Pro Glu Phe Glu Arg
           275                 280                 285
   aat gtg gag aat aca tct tgt ttc atg gtc cct ggt gtt ctt cct gat      972
   Asn Val Glu Asn Thr Ser Cys Phe Met Val Pro Gly Val Leu Pro Asp
       290                 295                 300
   act ttg aac aca gtc aga ata aga gtc aaa aca aat aag tta tgc tat     1020
   Thr Leu Asn Thr Val Arg Ile Arg Val Lys Thr Asn Lys Leu Cys Tyr
   305                 310                 315                 320
   gag gat gac aaa ctc tgg agt aat tgg agc caa gaa atg agt ata ggt     1068
   Glu Asp Asp Lys Leu Trp Ser Asn Trp Ser Gln Glu Met Ser Ile Gly
                   325                 330                 335
   aag aag cgc aat tcc aca ctc tac ata acc atg tta ctc att gtt cca     1116
   Lys Lys Arg Asn Ser Thr Leu Tyr Ile Thr Met Leu Leu Ile Val Pro
               340                 345                 350
   gtc atc gtc gca ggt gca atc ata gta ctc ctg ctt tac cta aaa agg     1164
   Val Ile Val Ala Gly Ala Ile Ile Val Leu Leu Leu Tyr Leu Lys Arg
           355                 360                 365
   ctc aag att att ata ttc cct cca att cct gat cct ggc aag att ttt     1212
   Leu Lys Ile Ile Ile Phe Pro Pro Ile Pro Asp Pro Gly Lys Ile Phe
       370                 375                 380
   aaa gaa atg ttt gga gac cag aat gat gat act ctg cac tgg aag aag     1260
   Lys Glu Met Phe Gly Asp Gln Asn Asp Asp Thr Leu His Trp Lys Lys
   385                 390                 395                 400
   tac gac atc tat gag aag caa acc aag gag gaa acc gac tct gta gtg     1308
   Tyr Asp Ile Tyr Glu Lys Gln Thr Lys Glu Glu Thr Asp Ser Val Val
                   405                 410                 415
   ctg ata gaa aac ctg aag aaa gcc tct cag tgatggagat aatttatttt       1358
   Leu Ile Glu Asn Leu Lys Lys Ala Ser Gln
               420                 425
   taccttcact gtgaccttga gaaga 1383
<210> 4
   <211> 426
   <212> PRT
   <213> human
<400> 4
   Met Glu Trp Pro Ala Arg Leu Cys Gly Leu Trp Ala Leu Leu Leu Cys
   1 5 10 15
   Ala Gly Gly Gly Gly Gly Gly Gly Gly Ala Pro Thr Glu Thr Gln Pro
   20 25 30
   Pro Val Thr Asn Leu Ser Val Ser Val Glu Asn Leu Cys Thr Val Ile
   35 40 45
   Trp Thr Trp Asn Pro Pro Glu Gly Ala Ser Ser Asn Cys Ser Leu Trp
   50 55 60
   Tyr Phe Ser His Phe Gly Asp Lys Gln Asp Lys Lys Ile Ala Pro Glu
   65 70 75 80
   Thr Arg Arg Ser Ile Glu Val Pro Leu Asn Glu Arg Ile Cys Leu Gln
   85 90 95
   Val Gly Ser Gln Cys Ser Thr Asn Glu Ser Glu Lys Pro Ser Ile Leu
   100 105 110
   Val Glu Lys Cys Ile Ser Pro Pro Glu Gly Asp Pro Glu Ser Ala Val
   115 120 125
   Thr Glu Leu Gln Cys Ile Trp His Asn Leu Ser Tyr Met Lys Cys Ser
   130 135 140
   Trp Leu Pro Gly Arg Asn Thr Ser Pro Asp Thr Asn Tyr Thr Leu Tyr
   145 150 155 160
   Tyr Trp His Arg Ser Leu Glu Lys Ile His Gln Cys Glu Asn Ile Phe
   165 170 175
   Arg Glu Gly Gln Tyr Phe Gly Cys Ser Phe Asp Leu Thr Lys Val Lys
   180 185 190
   Asp Ser Ser Phe Glu Gln His Ser Val Gln Ile Met Val Lys Asp Asn
   195 200 205
   Ala Gly Lys Ile Lys Pro Ser Phe Asn Ile Val Pro Leu Thr Ser Arg
   210 215 220
   Val Lys Pro Asp Pro Pro His Ile Lys Asn Leu Ser Phe His Asn Asp
   225 230 235 240
   Asp Leu Tyr Val Gln Trp Glu Asn Pro Gln Asn Phe Ile Ser Arg Cys
   245 250 255
   Leu Phe Tyr Glu Val Glu Val Asn Asn Ser Gln Thr Glu Thr His Asn
   260 265 270
   Val Phe Tyr Val Gln Glu Ala Lys Cys Glu Asn Pro Glu Phe Glu Arg
   275 280 285
   Asn Val Glu Asn Thr Ser Cys Phe Met Val Pro Gly Val Leu Pro Asp
   290 295 300
   Thr Leu Asn Thr Val Arg Ile Arg Val Lys Thr Asn Lys Leu Cys Tyr
   305 310 315 320
   Glu Asp Asp Lys Leu Trp Ser Asn Trp Ser Gln Glu Met Ser Ile Gly
   325 330 335
   Lys Lys Arg Asn Ser Thr Leu Tyr Ile Thr Met Leu Leu Ile Val Pro
   340 345 350
   Val Ile Val Ala Gly Ala Ile Ile Val Leu Leu Leu Tyr Leu Lys Arg
   355 360 365
   Leu Lys Ile Ile Ile Phe Pro Pro Ile Pro Asp Pro Gly Lys Ile Phe
   370 375 380
   Lys Glu Met Phe Gly Asp Gln Asn Asp Asp Thr Leu His Trp Lys Lys
   385 390 395 400
   Tyr Asp Ile Tyr Glu Lys Gln Thr Lys Glu Glu Thr Asp Ser Val Val
   405 410 415
   Leu Ile Glu Asn Leu Lys Lys Ala Ser Gln
   420 425
<210> 5
   <211> 30
   <212> PRT
   <213> artificial sequence
<220>
   <223> signal sequence of murine IL-3
<400> 5
   Met Val Leu Ala Ser Ser Thr Thr Ser Ile His Thr Met Leu Leu Leu
   1 5 10 15
   Leu Leu Met Leu Phe His Leu Gly Leu Gln Ala Ser Ile Ser
   20 25 30
<210> 6
   <211> 8
   <212> PRT
   <213> artificial sequence
<220>
   <223> N-terminal FLAG epitope-tag
<400> 6
   Asp Tyr Lys Asp Asp Asp Asp Lys
   1 5
<210> 7
   <211> 31
   <212> DNA
   <213> artificial sequence
<220>
   <223> Oligo 1478 5'
<400> 7
   agcttctaga acagaagttc agccacctgt g 31
<210> 8
   <211> 30
   <212> DNA
   <213> artificial sequence
<220>
   <223> Oligo 1480 5'
<400> 8
   aactccacct tctacaccac ctgatctaga 30
<210> 9
   <211> 5
   <212> PRT
   <213> unknown
<220>
   <220>
   <223> NR4 motif
<220>
   <221> MISC FEATURE
   <222> (3)..(3)
   <223> X may be any amino acid
<400> 9
   Trp Ser Xaa Trp Ser
   1               5
<210> 10
   <211> 27
   <212> PRT
   <213> unknown
<220>
   <223> N-terminal amino acid seuqence of mNR4 (major)
<220>
   <221> MISC FEATURE
   <222> (24)..(24)
   <223> X may be any amino acid
<400> 10
   Asp Tyr Lys Asp Asp Asp Asp Tyr Lys Asp Asp Asp Glu Ser Arg Thr
   1               5                   10                  15
   Glu Val Gln Pro Pro Val Thr Xaa Leu Ser Val
               20                  25
<210> 11
   <211> 27
   <212> PRT
   <213> unknown
<220>
   <223> N-terminal amino acid sequence of mNR4 (minor)
<220>
   <221> MISC_FEATURE
   <222> (24)..(24)
   <223> X may be any amino acid
<400> 11
   Ala Ser Ile Ser Ser Ser Asp Tyr Lys Asp Asp Asp Glu Ser Arg Thr
   1 5 10 15
   Glu Val Gln Pro Pro Val Thr Xaa Leu Ser Val
   20 25
<210> 12
   <211> 5
   <212> PRT
   <213> unknown
<220>
   <223> peptide motif found in many members of the haemopoietin receptor family
<400> 12
   Trp Ser Asp Trp Ser
   1 5

## Description

The present invention relates generally to a novel haemopoietin receptor or components or parts thereof as defined herein and to genetic sequences encoding same. The receptor molecules and their components and/or parts and the genetic sequences encoding same of the present invention are useful in the development of a wide range of agonists, antagonists, therapeutics and diagnostic reagents based on ligand interaction with its receptor.

Bibliographic details of the publications numerically referred to in this specification are collected at the end of the description. Sequence Identity Numbers (SEQ ID NOs.) for the nucleotide and amino acid sequences referred to in the specification are defined following the bibliography.

Throughout this specification and the claims which follow, unless the context requires otherwise, the word "comprise", or variations such as "comprises" or "comprising", will be understood to imply the inclusion of a stated integer or group of integers but not the exclusion of any other integer or group of integers.

The preferred haemopoietin receptor of the present invention is referred to herein as "NR4". The NR4 receptor interacts with IL-13 and is referred to herein as the IL-13 receptor or more particularly the IL-13 receptor α-chain (IL-13Rα). These terms are used interchangeably throughout the subject specification. The species from which a particular NR4 is derived is given in single letter abbreviation. For example, murine is "M" and human is "H", A recombinant form may have the prefix "r".

The rapidly increasing sophistication of recombinant DNA techniques is greatly facilitating research into the medical and allied health fields. Cytokine research is of particular importance, especially as these molecules regulate the proliferation, differentiation and function of a wide variety of cells. Administration of recombinant cytokines or regulating cytokine function and/or synthesis is increasingly becoming the focus of medical research into the treatment of a range of disease conditions.

Despite the discovery of a range of cytokines and other secreted regulators of cell function, comparatively few cytokines are directly used or targeted in therapeutic regimes. One reason , for this is the pleiotropic nature of many cytokines. For example, interleukin (IL)-11 is a functionally pleiotropic molecule (1,2), initially characterized by its ability to stimulate proliferation of the IL-6-dependent plasmacytoma cell line, T11 65 (3). Other biological actions of IL-11 include induction of multipotential haemopoietin progenitor cell proliferation (4,5,6), enhancement of megakaryocyte and platelet formation (7,8,9,10), stimulation of acute phase protein synthesis (11) and inhibition of adipocyte lipoprotein lipase activity (12, 13).

Interleukin-13 (IL-13) is another important cytokine which shares a number of structural characteristics with interleukin-4 (IL-4) [reviewed in 14 and 15]. The genes for IL-4 and IL-13 have a related intron/exon structure and are located close together on chromosome 5 in the human and the syntenic region of chromosome 11 in the mouse (14, 15). At the protein level, IL-4 and IL-13 share approximately 30% amino acid identity, including four cysteine residues. Biologically, IL-13 and IL-4 are also similar, being produced by activated T-cells and acting upon, for example, macrophages to induce differentiation and suppress the production of inflammatory cytokines. Additionally, human IL-13 may act as a co-stimulatory signal for B-cell proliferation and affect immunoglobulin isotype switching (14, 15). The diverse and pleiotropic function of IL-13 and other haemopoietic cytokines makes this group important to study, especially at the level of interaction of the cytokine with its receptors. Manipulation and control of cytokine receptors and of cytokine-receptor interaction is potentially very important in many therapeutic situations, especially where the target cytokine is functionally pleiotropic and it is desired to block certain functions of a target cytokine but not all functions.

Research into IL-13 and its receptor has been hampered due to the inability to clone genetic sequences encoding all or part of the IL-13 receptor. Zurawski et. al. (J. Biol. Chem., 1995, Vol. 270, No. 23, p13869-13878) discloses the identification of a primary binding protein for IL-13 in low numbers on haematopoietic cells. Obiri et al. (J. Biol. Chem., 1995, Vol. 270, No. 15, p8797-8804) also identifies an IL-13 binding protein on a variety of cells. The nucleotide sequences encoding these or any other IL-13 binding proteins were not disclosed. In accordance with the present invention, genetic sequences have now been cloned encoding the IL-13 receptor α-chain, a receptor subunit which is also shared with the IL-4 receptor. The availability of these genetic sequences permits the development of a range of therapeutic and diagnostic agents capable of modulating or monitoring IL-13 activity as well as the activity of cytokines related to IL-13 at the level of structure or function. In accordance with the present invention, an example of a cytokine related in structure and function to IL-13 is IL-4.

Disclosed herein is a nucleic acid molecule comprising a sequence of nucleotides encoding or complementary to a sequence encoding an haemopoietin receptor from an animal or a derivative of said receptor.

More particularly, an isolated nucleic acid molecule comprising a sequence of nucleotides encoding or complementary to a sequence encoding an animal haemopoietin receptor or a derivative thereof, said receptor capable of interaction with IL-13 or a derivative of IL-13 is disclosed.

In a related embodiment, an isolated nucleic acid molecule comprising a sequence of nucleotides encoding or complementary to a sequence encoding an animal haemopoietin receptor or a derivative thereof, wherein said receptor:
(i) is capable of interaction with IL-13 or its derivatives; and
(ii) is capable of interaction with a complex between IL-4 and IL-4 receptor α-chain, is disclosed.

In accordance with these embodiments, a derivative of IL-13 includes agonists, antagonists, antibodies and mimetics.

A nucleic acid molecule comprising a sequence of nucleotides encoding or complementary to a sequence encoding an animal IL-13 receptor α-chain or a derivative thereof is also disclosed

Also disclosed is a nucleic acid molecule comprising a sequence of nucleotides encoding or complementary to a sequence encoding a component of an animal IL-4 receptor or a derivative thereof.

Preferably, the animal is a mammal or a species of bird. Particularly, preferred animals include humans, laboratory test animals (e.g. primates, mice, rabbits, hamsters, guinea pigs), livestock animals (e.g. sheep, goats, horses, pigs, cows, donkeys), companion animals (e.g. dogs, cats), captive wild animals (e.g. foxes, kangaroos, dingoes) and poultry birds (e.g. chickens, geese, ducks) and game birds (e.g. emus, ostriches). Although the present invention is exemplified with respect to mice and humans, the scope of the subject invention extends to all animals and birds.

Accordingly, in a first aspect, the present invention provides an isolated nucleic acid molecule comprising a sequence of nucleotides which encodes or is complementary to a sequence which encodes a polypeptide, said polypeptide having an amino acid sequence
(i) as set forth in SEQ ID NO:2 or SEQ ID NO:4 or a part thereof which comprises the IL-13 binding portion of said amino acid sequence; or
(ii) having at least about 70% similarity to a sequence in (i); or
   said sequence of nucleotides having a nucleotide sequence
(iii) as set forth in SEQ ID NO:1 or SEQ ID NO:3 or a part thereof which comprises the sequence of said nucleotide sequence which encodes the IL-13 binding portion; or
(iv) having at least about 70% similarity to a sequence in (iii), or
(v) capable of hybridising to the nucleotide sequence as set forth in SEQ ID NO:1 or SEQ ID NO:3 or a complementary form thereof under high stringency conditions, using from at least 31% v/v to at least 50% v/v formamide and from at least 0.01 M to at least 0.15M salt for hybridisation, and at least 0.01 M to at least 0.15M for washing, or
(vi) which is a complementary sequence of a sequence of (iii) to (v),
wherein said polypeptide is capable of interaction with IL-13. Preferably said sequence of (v) comprises a nucleotide sequence coding for an amino acid
sequence having at least 70% similarity to the sequence set forth in SEQ ID NO:2 or SEQ ID NO:4. In a further preferred feature, the nucleic acid molecule encodes a haemopoietin receptor capable of interaction with IL-13, which interaction is capable of competitive inhibition by IL-4 in cells which express an IL-4 receptor α-chain. In a yet further aspect, the nucleic acid molecule encodes a polypeptide in the form of a fusion protein or in a soluble form or encodes a mature IL-13 receptor α-chain or a part thereof which comprises the IL-13 binding portion of said amino acid sequence.

The present invention is predicated in part on an ability to identify members of the haemopoietin receptor family on the basis of sequence similarity. Based on this approach, a genetic sequence was identified in accordance with the present invention which encodes a haemopoietin receptor. The expressed genetic sequence is referred to herein as "NR4". In accordance with the present invention, NR4 has an apparent molecular mass when synthesised by transfected COS cells of from about 50,000 to about 70,000 daltons, and more preferably from about 55,000 to about 65,000 daltons. NR4 binds to IL-13 specifically and with low affinity and is considered, therefore, to be an IL-13 receptor α-chain. Accordingly, the terms "NR4" and "IL-13 receptor α-chain" (or "IL-13 Rα") are used interchangeably throughout the subject specification. Furthermore, IL-13 binding to its receptor has been found to be competitively inhibited by IL-4 or a component thereof in cells which express the IL-4 receptor α-chain and this may provide a method for controlling IL-13-receptor interaction and will also provide a basis for the preparation and construction of mimetics.

Also disclosed is a nucleic acid molecule comprising a sequence of nucleotides encoding IL-13 receptor α-chain having an amino acid sequence as set forth in SEQ ID NO:2 or 4 or having at least about 50%, preferably at least 60% similarity to all or part thereof. In preferred aspects of the invention, the percentage similarity to any one of SEQ ID NOs 1 to 4 or a part thereof is at least about 80-85% and more preferably at least about 90-95% or greater. The reference to all or part of a sequence is intended to include defining a hybrid molecule comprising parts of two receptors. It is not intended to encompass single amino acids.

A nucleic acid molecule comprising a sequence of nucleotides encoding the IL-13 receptor α-chain and having a nucleotide sequence substantially as set forth in SEQ ID NO:1 or 3 or having at least about 50%, preferably at least about 60% is also disclosed.

Accordingly, the present invention extends to the sequence of nucleotides set forth in SEQ ID NO:1 or 3 or the sequence of amino acids set forth in SEQ ID NO:2 or 4 or single or multiple nucleotide or amino acid substitutions, deletions and/or additions thereto as defined in the claims.

Nucleic acid molecules capable of hybridising under low stringency conditions to the nucleotide sequence set forth in SEQ ID NO:1 or 3 or a complementary form thereof are also disclosed.

The present invention extends to recombinant haemopoietin receptors and in particular recombinant NR4 and recombinant hybrids containing NR4 as described in the claims. Preferred recombinant polypeptides interact with IL-13 with low affinity and even more preferably with high affinity.

A polypeptide having at least two of the following:
(i) comprises an amino acid sequence substantially as set forth in SEQ ID NO:2 or SEQ ID NO:4 or having at least about 50% similarity to all or part thereof;
(ii) is encoded by a nucleotide sequence substantially as set forth in SEQ ID NO:1 or SEQ ID NO:3 or having at least about 50% similarity to all or part thereof;
(iii) interacts with IL-13 or its derivatives with at least low affinity; and
(iv) has a molecular weight of from about 50,000 to about 70,000 daltons as determined by Western blot analysis when expressed in COS cells, is disclosed.

A polypeptide having at least three of the following characteristics:
(i) comprises an amino acid sequence substantially as set forth in SEQ ID NO:2 or SEQ ID NO:4 or having at least about 50% similarity to all or part thereof;
(ii) is encoded by a nucleotide sequence substantially as set forth in SEQ ID NO:1 or SEQ ID NO:3 or having at least about 50% similarity to all or part thereof;
(iii) interacts with IL-13 or its derivatives with at least low affinity:
(iv) has a molecular weight of from about 50,000 to about 70,000 daltons as determined by Western blot analysis when expressed in COS cells;
(v) comprises an amino acid sequence derived from IL-4 receptor α-chain; and
(vi) is capable of interaction with IL-13 which is competitively inhibited by IL-4 in cells which express an IL-4 receptor α-chain, is also disclosed.

Thus the present invention provides a recombinant polypeptide comprising a sequence of amino acids (i) as set forth in SEQ ID NO:2 or SEQ ID NO:4 or a part thereof which comprises the IL-13 binding portion of said amino acid sequence; or (ii) having at least about 70% similarity to a sequence in (i) or encoded by a nucleotide sequence (iii) as set forth in SEQ ID NO:1 or SEQ ID NO:3 or a part thereof which comprises the sequence of said nucleotide sequence which encodes the IL-13 binding portion; or (iv) having at least about 70% similarity to a sequence in (iii) or a complementary sequence thereof, said polypeptide being capable of interaction with IL-13. Preferably the interaction with IL-13 is with low affinity, especially preferably with medium to high affinity. In a particularly preferred embodiment said polypeptide has a molecular weight of from about 50,000 to about 70,000 daltons as determined by Western blot analysis when expressed in COS cells. In a further preferred aspect, the polypeptide is capable of interaction with IL-13 which is competitively inhibited by IL-4 in cells which express an IL-4 receptor α-chain.

Reference herein to "recombinant haemopoietin receptor", "NR4", "IL-13 receptor" or "IL-13" receptor α-chain" includes reference to derivatives thereof such as parts, fragments, portions, homologues, hybrids or analogues thereof. The derivatives may be functional or not or may be non-functional but immunologically interactive with antibodies to all or part of the receptor. Derivatives of the receptor also cover agonists or antagonists of receptor-ligand interaction. Function is conveniently defined by an ability of NR4 to interact with IL-13 or its derivatives or for soluble NR4 to compete with IL-13-induced activities of certain cells.

Particularly preferred derivatives contemplated by the present invention include derivatives of IL-13 receptor α-chain which are capable of binding IL-13 with high affinity or with IL-13 and IL-4 with high affinity; derivatives also encompass chimeric molecules such as between IL-13 receptor α-chain and, for example, IL-4 receptor α-chain which also bind IL-13 with high affinity.

Other fusion or chimeric molecules contemplated by the present invention include those between NR4 and members of the haemopoietin receptor family, receptor tyrosine kinases. TNF/NGF receptors and G protein-coupled receptors. For example, chimeras may be between NR4 and IL-13 binding protein, IL-4 receptor α-chain, IL-2 receptor γ-chain or receptors for other cytokines involved or implicated in asthma and allergy such as IL-5. Other important chimeras include NR4 and immunoglobulins or other molecules which allow targeting of NR4 to particular cells or tissues, NR4 and toxins and NR4 and growth factors.

Thus, in a preferred aspect, the polypeptide is in the form of a fusion protein. In a further preferred aspect the polypeptide is a mature IL-13 receptor α-chain or a part thereof which comprises the IL-13 binding portion of said amino acid sequence. In yet further preferred aspects the present invention provides a hybrid haemopoietin receptor capable of interaction with at least two cytokines wherein at least one of said cytokines is IL-13 and wherein said hybrid receptor comprises an amino acid sequence which includes all of the amino acid sequence set forth in SEQ ID NO:2 or SEQ ID NO:4 or a part thereof which comprises the IL-13 binding portion of said amino acid sequence or a hybrid haemopoietin receptor capable of high affinity interaction with at least one cytokine wherein at least one of said cytokines is IL-13 and wherein said hybrid receptor comprises an amino acid sequence which includes all of the amino acid sequence set forth in SEQ ID NO:2 or SEQ ID NO:4 or a part thereof which comprises the IL-13 binding portion of said amino acid sequence.

Preferably said receptor is capable of interaction with a cytokine selected from IL-4, IL-2, IL-5, IL-7, IL-9 and IL-15. The present invention also provides a fusion protein comprising a first portion capable of interaction with IL-13, wherein said first portion is encoded by a nucleic acid molecule of the invention as hereinbefore described and a second portion derived from a haemopoietin receptor, a receptor tyrosine kinase, a TNF/NGF receptor or a G protein coupled receptor, wherein preferably the second portion comprises all or a functional portion of IL-13 binding protein, IL-4 receptor α-chain, IL-2 receptor γ-chain or a receptor for a cytokine implicated in asthma or allergy.

Reference herein to a low stringency at 42°C includes and encompasses from at least about 1% v/v to at least about 15% v/v formamide and from at least about 1M to at least about 2M salt for hybridisation, and at least about 1 M to at least about 2M salt for washing conditions. Alternative stringency conditions may be applied where necessary, such as medium stringency, which includes and encompasses from at least about 16% v/v to at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01 M to at least about 0.15M salt for hybridisation, and at least about 0.01 M to at least about 0.15M salt for washing conditions.

Also disclosed is a nucleic acid molecule comprising a sequence of nucleotides which encodes or is complementary to a sequence which encodes an IL-13 receptor α-chain, said nucleic acid molecule having a nucleotide sequence substantially as set forth in SEQ ID NO:1 or 3 or a nucleic acid molecule which encodes a structurally similar IL-13 receptor α-chain or a derivative thereof and which is capable of hybridising to the nucleotide sequence substantially as set forth in SEQ ID NO:1 or 3 or a complementary form thereof under low stringency conditions.

A nucleic acid molecule comprising a sequence of nucleotides which encodes or is complementary to a sequence which encodes the IL-13 receptor α-chain having an amino acid sequence substantially as set forth in SEQ ID NO:2 or 4 or comprises a nucleotide sequence coding for an amino acid sequence having at least about 50% similarity to the sequence set forth in SEQ ID NO:2 or 4 and is capable of hybridising to the sequence set forth in SEQ ID NO:1 or 3 under low stringency conditions is also disclosed.

The nucleic acid molecules contemplated by the present invention are generally in isolated form and may be single or double stranded, linear or closed circle DNA (e.g. genomic DNA), cDNA or mRNA or combinations thereof such as in the form of DNA:RNA hybrids. In a particularly preferred embodiment, the nucleic acid molecules are in vectors and most preferably expression vectors to enable expression in a suitable host cell. Particularly useful host cells include prokaryotic cells, mammalian cells, yeast cells and insect cells. The cells may also be in the form of a cell line.

Accordingly there is provided an expression vector comprising a nucleic acid molecule encoding the IL-13 receptor α-chain as hereinbefore described, said expression vector capable of expression in a particular host cell.

Preferably, the percentage similarity to any one of the ID NOs 1 to 4 or a part thereof is at least about 80-85% and still even more preferably at least about 90-95% or greater.

The recombinant polypeptide contemplated by the present invention includes, therefore, components, parts, fragments, derivatives, homologues or analogues of the IL-13 receptor α-chain according to the claims and is preferably encoded by a nucleotide sequence as set forth in SEQ ID NO:1 or 3 or a molecule having at least about 70% similarity to all or part thereof or a molecule capable of hybridising to the nucleotide sequence set forth in SEQ ID NO:1 or 3 or a complementary form thereof under high stringency conditions as set forth in the claims. The recombinant molecule may be glycosylated or non-glycosylated. When in glycosylated form, the glycosylation may be substantially the same as naturally occurring IL-13 receptor α-chain or may be a modified form of glycosylation. Altered or differential glycosylation states may or may not affect binding activity of the IL-13 receptor α-chain.

The recombinant IL-13 receptor α-chain may be in soluble form or may be expressed on a cell surface or conjugated or fused to a solid support or another molecule.

The present invention further contemplates a method for producing a recombinant polypeptide of the invention as set forth hereinbefore, said method comprising culturing cells comprising the genetic constructs of the present invention for a time and under conditions sufficient to express the nucleic acid molecule in said genetic construct to produce a recombinant polypeptide and isolating said recombinant polypeptide.

The present invention further extends to isolated animal cells comprising the genetic construct of the invention described herein and which express the above-mentioned recombinant polypeptides produced by the above described method.

The present invention as defined in the claims encompasses chemical analogues of the recombinant IL-13 receptor α-chain.

As stated above, the present invention encompasses a range of derivatives of NR4. Derivatives include fragments, parts, portions, mutants, hybrids (including fusion and chimeric molecules), homologues and analogues of the NR4 polypeptide and corresponding genetic sequence. In one preferred embodiment, the derivatives bind IL-13 with high affinity. Other preferred derivatives act as agonists, antagonist or mimetics. Derivatives also include single or multiple amino acid substitutions, deletions and/or additions to NR4 or single or multiple nucleotide substitutions, deletions and/or additions to the genetic sequence encoding NR4. "Additions" to amino acid sequences or nucleotide sequences include fusions with other peptides, polypeptides or proteins or fusions to nucleotide sequences. Reference herein to "NR4" includes reference to all derivatives thereof including functional derivatives or "NR4" immunologically interactive derivatives. The present invention also extends to hybrid molecules, such as between murine or human NR4 or derivatives thereof. A particularly preferred hybrid comprises NR4 and IL-4 receptor α-chain.

Analogues of NR4 contemplated herein include, but are not limited to, modification to side chains, incorporating of unnatural amino acids and/or their derivatives during peptide, polypeptide or protein synthesis and the use of crosslinkers and other methods which impose conformational constraints on the proteinaceous molecule or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 1.

Crosslinkers can be used, for example, to stabilise 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH).

In addition, peptides can be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

These types of modifications may be important to stabilise NR4 if administered to an individual or for use as a diagnostic reagent.

The present invention further encompasses chemical analogues of NR4 capable of acting as antagonists or agonists of NR4 or which can act as functional analogues of NR4. Chemical analogues may not necessarily be derived from NR4 but may share certain conformational similarities. Alternatively, chemical analogues may be specifically designed to mimic certain physiochemical properties of NR4. Chemical analogues may be chemically synthesised or may be detected following, for example, natural product screening.

The identification of NR4 permits the generation of a range of therapeutic molecules capable of modulating expression of NR4 or modulating the activity of NR4. Such modulator include agonists and antagonists of NR4 gene expression or NR4 protein activity. Antagonists of NR4 gene expression include antisense molecules, ribozymes and co-suppression molecules. Agonists include molecules which increase promoter ability or interfere with negative regulatory mechanisms. Agonists of NR4 protein include antibodies, ligands and mimetics. Antagonists of NR4 include antibodies and inhibitor peptide fragments. Where a cell co-expresses NR4 and IL-4 receptor α-chain, agonists and antagonists may target the IL-4 receptor α-chain.

**TABLE 1**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | | Chexa L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylieucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylomithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvatine | Mval | L-N-methylhomophenylalanine | Nmhphe |
| N-(N-(2,2-diphenylethyl) carbamylmethyl)glycine | Nnbhm | N-(N-(3,3-diphenylpropyl) carbamylmethyl)glycine | Nnbhe |
| 1-carboxy-1-(2,2-diphenyl-ethylamino)cyclopropane | Nmbc | | |

Other derivatives encompassed by the present invention include a range of glycosylation variants from a completely unglycosylated molecule to a modified glycosylated molecule. Altered glycosylation patterns may result from expression of recombinant molecules in different host cells.

Modulators as described herein may be used in a method for modulating expression of the NR4 gene in a human, said method comprising contacting the NR4 gene encoding NR4 with an effective amount of a modulator of NR4 expression for a time and under conditions sufficient to up-regulate or down-regulate or otherwise modulate expression of NR4. A nucleic acid molecule encoding NR4 or a derivative thereof may also be introduced into a cell to enhance or alter NR4 related activities of that cell including replacing an endogenous NR4 gene sequence which may, for example, be defective or carry one or more undesired mutations. Conversely, NR4 antisense sequences (or sense sequences for co-suppression) such as oligonucleotides may be introduced to decrease NR4-related activies of any cell expressing the endogenous NR4 gene. Ribozymes may also be used.

NR4 activity may be modulated in a human by a method comprising administering to said mammal a modulating effective amount of a molecule for a time and under conditions sufficient to increase or decrease NR4 activity. The molecule may be a proteinaceous molecule or a chemical entity and may also be a derivative of NR4 or its ligand or a chemical analogue or truncation mutant of NR4 or its ligand.

For example, IL-13 and IL-4 have been impliciated in the modulation of immune responses and in the production of IgE which is the immunoglobulin isotype associated with allergic or atopic diseases such as asthma. Modulating interactions between IL-13/IL-4 and their receptors may be important in treating inflammatory conditions such as allergic conditions. Elevated levels of IL-4/IL-13 and IgE are also important in diseases such as nephrotic syndrome, vernal and keratoconjunctivitis. Other diseases, the treatment of which is contemplated herein include bronchial asthma, perennial rhinitis and atopic dermatitis. Other disease conditions for which modulation of IL-13-receptor interaction may be important includes those conditions where IL-13 induces cytokine formation which in turn are involved in onset, progression and/or severity of diseases. Similarly, modulating IL-4-receptor interaction may also be important in controlling disease conditions. For example, some cancers may be exacerbated by the cytokine IL-13 or IL-4 which induce repressive immune effects or effector molecules which in turn reduce the body's ability to respond to the growth of the cancers.

For the above described methods a pharmaceutical composition comprising NR4 or a derivative thereof or a modulator of NR4 expression or NR4 activity e.g. a recombinant haemopoietin receptor as hereinbefore described or a ligand (e.g. IL-13) binding portion thereof or a ligand (e.g. IL-13) to the recombinant haemopoietin receptor as hereinbefore described and one or more pharmaceutically acceptable carriers and/or diluents may be used. These components are referred to as the "active ingredients".

In this regard there is provided a pharmaceutical composition comprising a recombinant polypeptide as hereinbefore described and one or more pharmaceutically acceptable carriers and/or diluents.

Such compositions may be used in a method of treatment of an animal comprising administering to said animal a treatment effective amount of a recombinant haemopoietin receptor as hereinbefore described or a ligand binding portion thereof or a ligand (e.g. IL-13) to said haemopoietin receptor for a time and under conditions sufficient for said treatment to be substantially effected or the conditions to be substantially ameliorated.

Thus in a further aspect the present invention provides a recombinant polypeptide of the invention as hereinbefore described for use as a medicament, preferably for treating asthma, allergy or a condition exacerbated by IgE production.

The pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as licithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 ug and 2000 mg of active compound.

The tablets, troches, pills, capsules and the like may also contain the components as listed hereafter: A binder such as gum, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

The present invention also extends to forms suitable for topical application such as creams, lotions and gels.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 0.5 µg to about 2000 mg. Expressed in proportions, the active compound is generally present in from about 0.5 µg to about 2000 mg/ml of carrier. In the case of compositions containing supplementary active ingredients, the dosages are determined by reference to the usual dose and manner of administration of the said ingredients.

The pharmaceutical composition may also comprise genetic molecules such as a vector capable of transfecting target cells where the vector carries a nucleic acid molecule capable of modulating NR4 expression or NR4 activity. The vector may, for example, be a viral vector. Thus in a further aspect the present invention provides a pharmaceutical composition comprising a nucleic acid molecule of the invention as described hereinbefore and one or more pharmaceutically acceptable carriers and/or diluents.

Still another aspect of the present invention is directed to antibodies to NR4 and its derivatives of the invention as described hereinbefore. Accordingly the present invention provides an antibody specific to the recombinant polypeptide of the invention as described hereinbefore. Such antibodies may be monoclonal or polyclonal and may be selected from naturally occurring antibodies to NR4 or may be specifically raised to NR4 or derivatives thereof. In the case of the latter, NR4 or its derivatives may first need to be associated with a carrier molecule. The antibodies and/or recombinant NR4 or its derivatives of the present invention are particularly useful as therapeutic or diagnostic agents. Accordingly, the present invention further provides antibodies of the invention for use as a medicament.

For example, NR4 and its derivatives can be used to screen for naturally occurring antibodies to NR4. These may occur, for example in some autoimmune diseases. Alternatively, specific antibodies can be used to screen for NR4. Technique for such assays are well known in the art and include, for example, sandwich assays and ELISA. Knowledge of NR4 levels and/or IL-13 levels may be important for diagnosis of certain cancers or a predisposition to cancers or for monitoring certain therapeutic protocols. In particular, it may be important to monitor an IgE response or levels of IL-13 or IL-4 or both which in turn have an effect on the immune system.

Antibodies to NR4 of the present invention may be monoclonal or polyclonal. Alternatively, fragments of antibodies may be used such as Fab fragments. Furthermore, the present invention extends to recombinant and synthetic antibodies and to antibody hybrids. A "synthetic antibody" is considered herein to include fragments and hybrids of antibodies. The antibodies of this aspect of the present invention are particularly useful for immunotherapy and may also be used as a diagnostic tool for assessing the receptor or receptor-ligand interaction or monitoring the program of a therapeutic regimen.

For example, specific antibodies can be used to screen for NR4 proteins. The latter would be important, for example, as a means for screening for levels of NR4 in a cell extract or other biological fluid or purifying NR4 made by recombinant means from culture supernatant fluid. Techniques for the assays contemplated herein are known in the art and include, for example, sandwich assays and ELISA.

It is within the scope of this invention to include any second antibodies (monoclonal, polyclonal or fragments of antibodies or synthetic antibodies) directed to the first mentioned antibodies discussed above. Both the first and second antibodies may be used in detection assays or a first antibody may be used with a commercially available anti-immunoglobulin antibody. An antibody as contemplated herein includes any antibody specific to any region of NR4.

Both polyclonal and monoclonal antibodies are obtainable by immunization with the receptor and either type is utilizable for immunoassays. The methods of obtaining both types of sera are well known in the art. Polyclonal sera are less preferred but are relatively easily prepared by injection of a suitable laboratory animal with an effective amount of NR4, or antigenic parts thereof, collecting serum from the animal, and isolating specific sera by any of the known immunoadsorbent techniques. Although antibodies produced by this method are utilizable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Antibodies as described herein may be used in a method for detecting NR4 in a biological sample from a subject said method comprising contacting said biological sample with an antibody specific for NR4 or its derivatives or homologues for a time and under conditions sufficient for an antibody-NR4 complex to form, and then detecting said complex.

The presence of NR4 may be accomplished in a number of ways such as by Western blotting and ELISA procedures. A wide range of immunoassay techniques are available as can be seen by reference to US Patent Nos. 4,016,043, 4, 424,279 and 4,018,653. These, of course, includes both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist, and may be used. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody. These techniques are well known to those skilled in the art, including any minor variations as will be readily apparent. The sample is one which might contain NR4 including cell extract, tissue biopsy or possibly serum, saliva, mucosal secretions, lymph, tissue fluid and respiratory fluid. The sample is, therefore, generally a biological sample comprising biological fluid, cell extract, bone marrow or lymph, tissue extract (e.g. from kidney, liver, spleen, etc), fermentation fluid and supernatant fluid such as from a cell culture and cell conditioned medium.

In the typical forward sandwich assay, a first antibody having specificity for the NR4 or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any subunit present in the antibody. Following the incubation period, the antibody subunit solid phase is washed and dried and incubated with a second antibody specific for a portion of the hapten. The second antibody is linked to a reporter molecule which is used to indicate the binding of the second antibody to the hapten.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules. In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternatively, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

Another form of assay involves cells capable of expressing NR4 and IL-4 receptor α-chain. For example, if IL-4 receptor α-chain and NR4 are co-expressed on cells, such as COS cells, then IL-13 binds to NR4 with a high affinity in the presence of IL-4.

Although not intending to limit the present invention to any one theory or mode of action, when NR4 and the IL-4 receptor are expressed in the same cell, they contribute to the formation of both IL-4 and IL-13 receptors. In the case of IL-4, binding occurs first through the IL-4 receptor α-chain and then NR4 interacts with this complex. In the case of IL-13, binding occurs first to NR4 and then IL-4 receptor α-chain interacts with the complex to form a high affinity receptor capable of signal transduction. The consequences of co-expression of NR4 and IL-4 receptor α-chain is that IL-4 and 1L-13 can compete with each other for binding to the IL-4 receptor α-chain and NR4.

Based on this behaviour, it would appear that any protein or small molecule that prevented IL-4 or IL-13 forming cell surface complexes containing both receptor components may be antagonistic. Such molecules may prevent interaction of the cytokine with its low affinity receptor. For example, soluble IL-13BP can prevent IL-13 interaction with NR4. Likewise, soluble IL-4 receptor α-chain can prevent binding of IL-4 to cell surface IL-4 receptor α-chain. These reagents would be antagonists that were specific for IL-4 or IL-13.

By extension, because of its very low affinity, soluble NR4 is a very inefficient IL-13 antagonist. If a soluble NR4 mutant is selected that now binds to IL-4 and also binds to IL-13 with higher affinity, this would be a useful antagonist of both IL-4 and IL-13.

An alternative to use of soluble receptor, is to generate a panel of monoclonal antibodies to NR4. If an antibody is obtained which prevents interaction of NR4 with the IL-4 receptor α-chain, a critical event in formation of both functional IL-4 receptor and functional IL-13 receptors, then again the action of both cytokines is inhibited.

Antibodies of the invention which inhibit the interaction of a polypeptide as described hereinbefore with the IL-4 receptor α-chain form preferred aspects of the invention.

The level of IL-4 in a biological sample may be monitored by a method comprising incubating said biological sample with cells which express NR4 and IL-4 receptor α-chain together with an effective amount of IL-13 to competitively inhibit IL-4 binding to its receptor and determining the extent of competitive inhibition.

In a related method the level of IL-13 in a biological sample may be monitored by a method comprising incubating said biological sample with cells which express NR4 and IL-4 receptor α-chain together with an effective amount of IL-4 to competitively inhibit IL-13 binding to its receptor and determining the extent of competitive inhibition.

Preferably, the cytokines are labelled with a reporter molecule as described above.

The biological sample includes but is not limited to blood, serum, plasma, tissue fluid, tissue extract, lymph, T cells or extracts thereof, culture supernatant and conditioned medium.

The NR4 gene or its derivatives may also be detected by genetic assays such as involving PCR analysis. Alternative methods or methods used in conjunction include direct nucleotide sequencing or mutation scanning such as single stranded conformation polymorphisms analysis (SSCP) as specific oligonucleotide hybridisation, as methods such as direct protein truncation tests. Such genetic tests may be important, for example, in genetic screening of animals (e.g. humans) for non-expression or substantial absence of expression or expression of mutant forms of NR4 leading to disease conditions.

The nucleic acid molecules of the present invention may be DNA or RNA. When the nucleic acid molecule is in DNA form, it may be genomic DNA or cDNA. RNA forms of the nucleic acid molecules of the present invention are generally mRNA.

Although the nucleic acid molecules of the present invention are generally in isolated form, they may be integrated into or ligated to or otherwise fused or associated with other genetic molecules such as vector molecules and in particular expression vector molecules. Vectors and expression vectors are generally capable of replication and, if applicable, expression in one or both of a prokaryotic cell or a eukaryotic cell. Preferably, prokaryotic cells include *E. coli, Bacillus sp* and *Pseudomonas sp*. Preferred eukaryotic cells include yeast, fungal, mammalian and insect cells.

Thus, a genetic construct comprising a vector portion and a mammalian and more particularly a human NR4 gene portion, which NR4 gene portion is capable of encoding an NR4 polypeptide or a functional or immunologically interactive derivative thereof is disclosed.

Preferably, the NR4 gene portion of the genetic construct is operably linked to a promoter on the vector such that said promoter is capable of directing expression of said NR4 gene portion in an appropriate cell.

In addition, the NR4 gene portion of the genetic construct may comprise all or part of the gene fused to another genetic sequence such as a nucleotide sequence encoding glutathione-S-transferase or part thereof or a cytokine or another haemopoietin receptor. Hybrid receptor molecules are particularly useful in the development of multi functional therapeutic and diagnostic agents.

The present invention extends to such genetic constructs and to prokaryotic or eukaryotic cells comprising same. Thus in a further aspect the present invention provides a genetic construct comprising a nucleic acid molecule of the invention as hereinbefore described operably linked to a promoter capable of directing expression of said nucleic acid molecule in a host cell.

The present invention also extends to any or all derivatives of NR4 including mutants, part, fragments, portions, homologues and analogues or their encoding genetic sequence including single or multiple nucleotide or amino acid substitutions, additions and/or deletions to the naturally occurring nucleotide or amino acid sequence as encompassed by the claims.

The NR4 and its genetic sequence of the present invention will be useful in the generation-of a range of therapeutic and diagnostic reagents and will be especially useful in the detection of a corresponding ligand. For example, recombinant NR4 may be bound or fused to a reporter molecule capable of producing an identifiable signal, contacted with a biological sample putatively containing a ligand and screening for binding of the labelled NR4 to the ligand. Alternatively, labelled NR4 may be used to screen expression libraries of putative ligand genes or functional parts thereof.

In another embodiment, the NR4 is first immobilised. According to this embodiment, there is provided a method comprising contacting a biological sample containing a putative ligand with said haemopoietin receptor or a ligand binding portion thereof immobilised to a solid support for a time and under conditions sufficient for a complex to form between said receptor and said ligand if said ligand is present in said biological sample, eluting bound ligand and isolating same.

Soluble NR4 polypeptides as well as various hybrids are also contemplated to be useful in the treatment of disease, injury or abnormality in the nervous system, e.g. in relation to central or peripheral nervous system to treat Cerebral Palsy, trauma induced paralysis, vascular ischaemia associated with stroke, neuronal tumours, motoneurone disease, Parkinson's disease, Huntington's disease, Alzheimer's disease, Multiple Sclerosis, peripheral neuropathies associated with diabetes, heavy metal or alcohol toxicity, renal failure and infectious diseases such as herpes, rubella, measles, chicken pox, HIV or HTLV-1. The NR4 polypeptides and hybrids may also be important for regulating cytokine activity and/or modulating haemopoiesis. They are also important for treating allergic or atopic conditions as well as other inflammatory conditions such as rheumatoid arthritis.

As stated above, the NR4 or its ligand or their functional derivatives may be provided in a pharmaceutical composition together with one or more pharmaceutically acceptable carriers and/or diluents. In addition, a method of treatment comprising the administration of an effective amount of NR4 is disclosed herein. Antagonists and agonists of NR4 and/or its ligand may be used in therapeutic compositions and methodologies.

NR4 or its functional derivatives may be used in the manufacture of a medicament for the treatment of NR4 mediated conditions defective or deficient.

The present invention is further described by the following non-limiting Figures and Examples.

In the Figures:

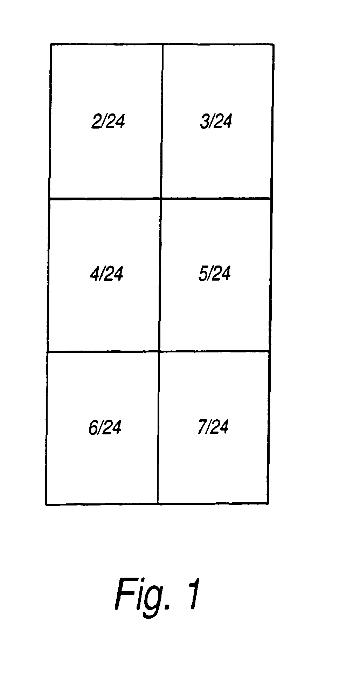

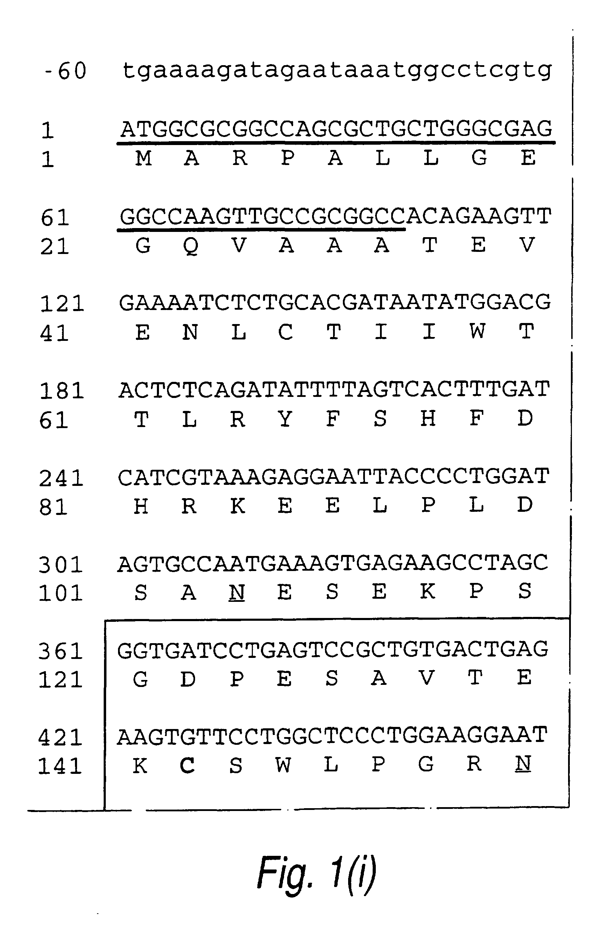

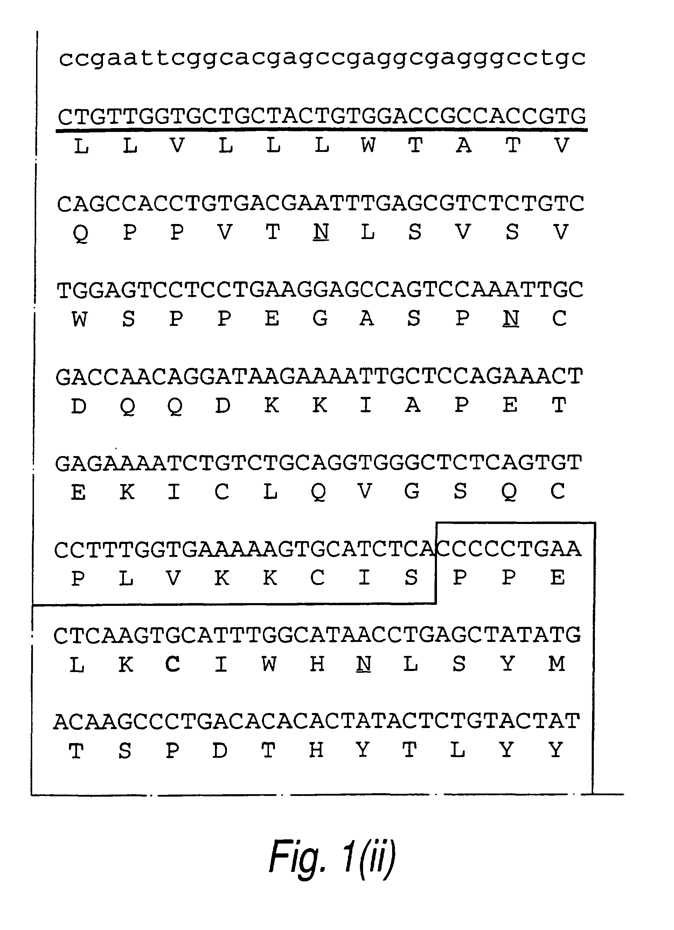

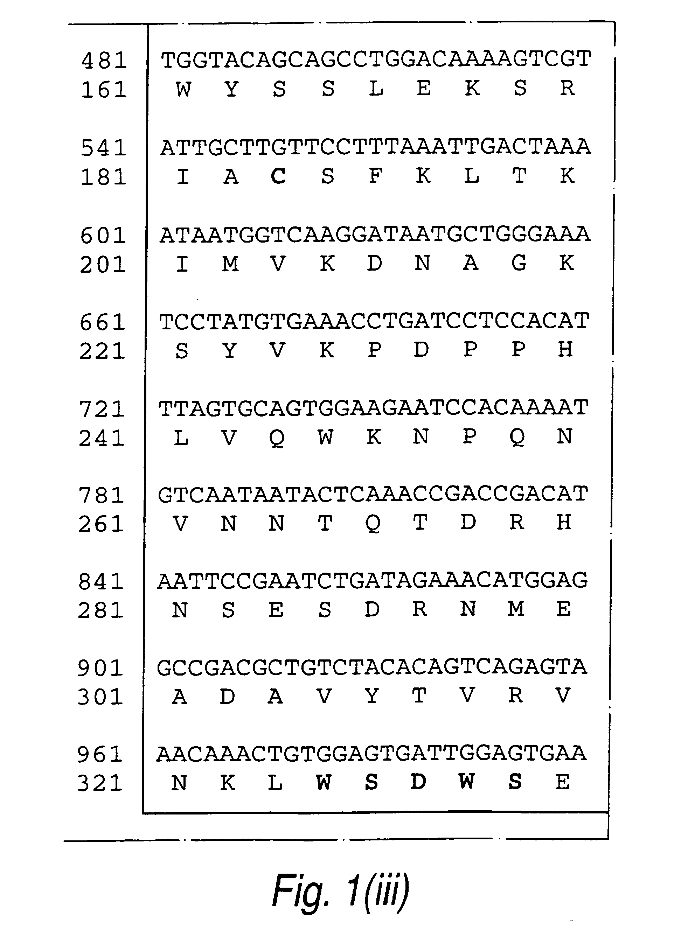

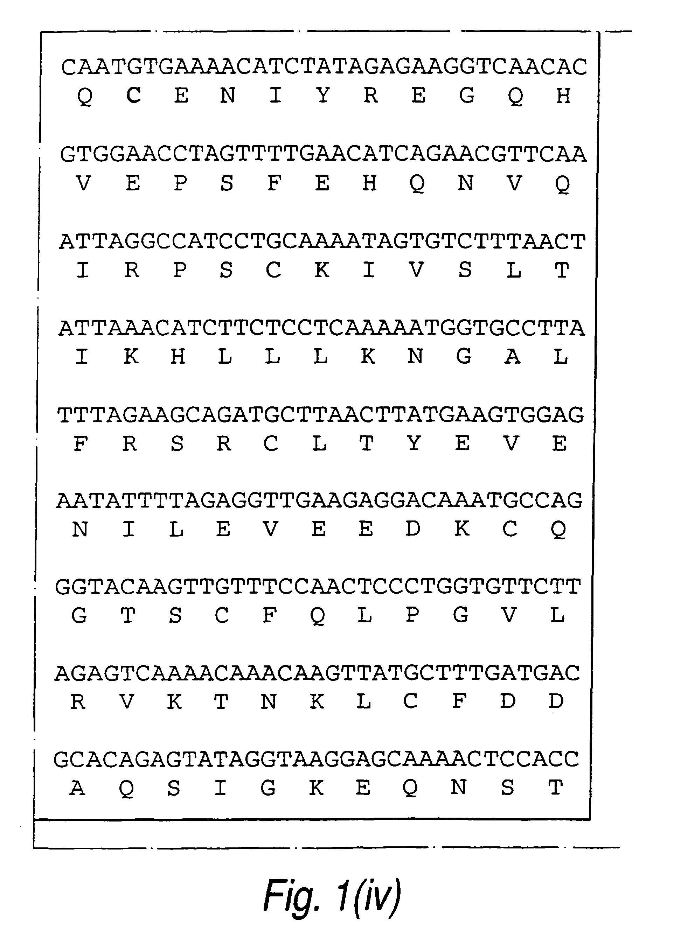

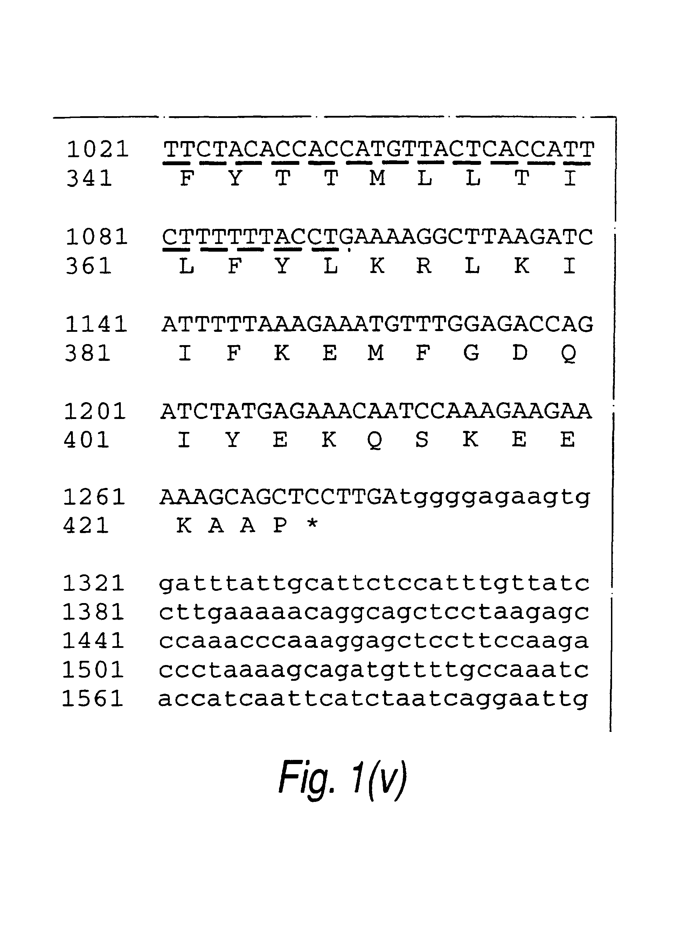

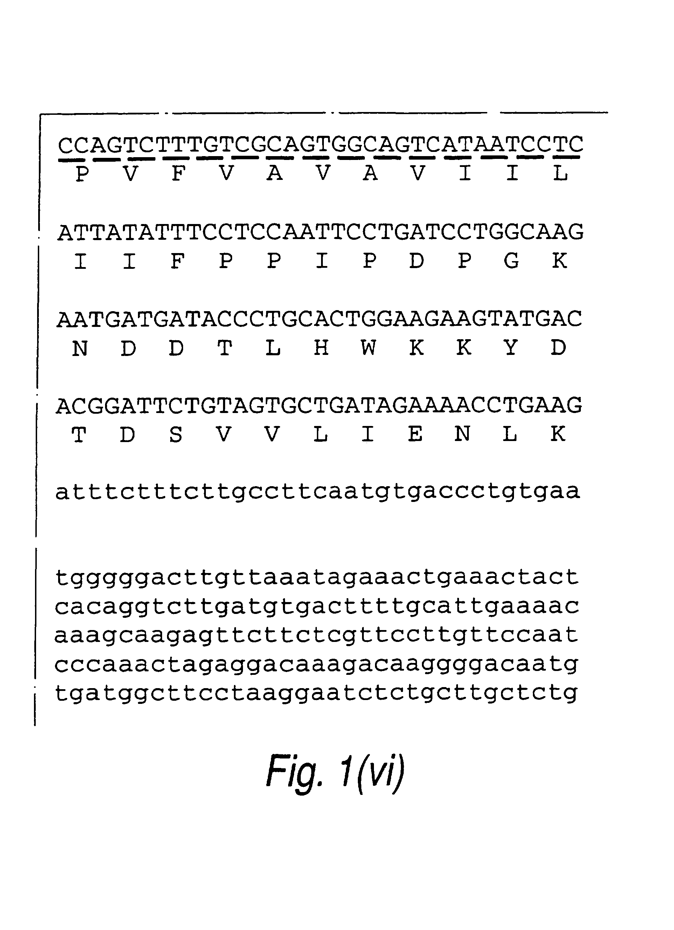

**Figure 1** is a representation of the nucleotide [SEQ ID NO:1] and predicted amino acid [SEQ ID NO:2] sequence of murine NR4. The untranslated region is shown in lower case and the translated region in upper case. The conventional one-letter code for amino acids is employed, potential asparagine linked glycosylation sites are underlined and the conserved cysteine residues and WSXWS [SEQ ID NO: 9] motif of haemopoietin receptor family members are shown in bold. The predicted signal sequence is underlined in bold while the transmembrane domain is underlined with dashes. The sequence shown is a composite derived from the analysis of 8 cDNA clones derived from 3 libraries. The 5'-end of the sequence (nucleotides -60 to 351) is derived from a single cDNA clone but is also present in genomic DNA clones that have been isolated. Boxed region - typical haemopoietin receptor domain, amino acids 118-340.

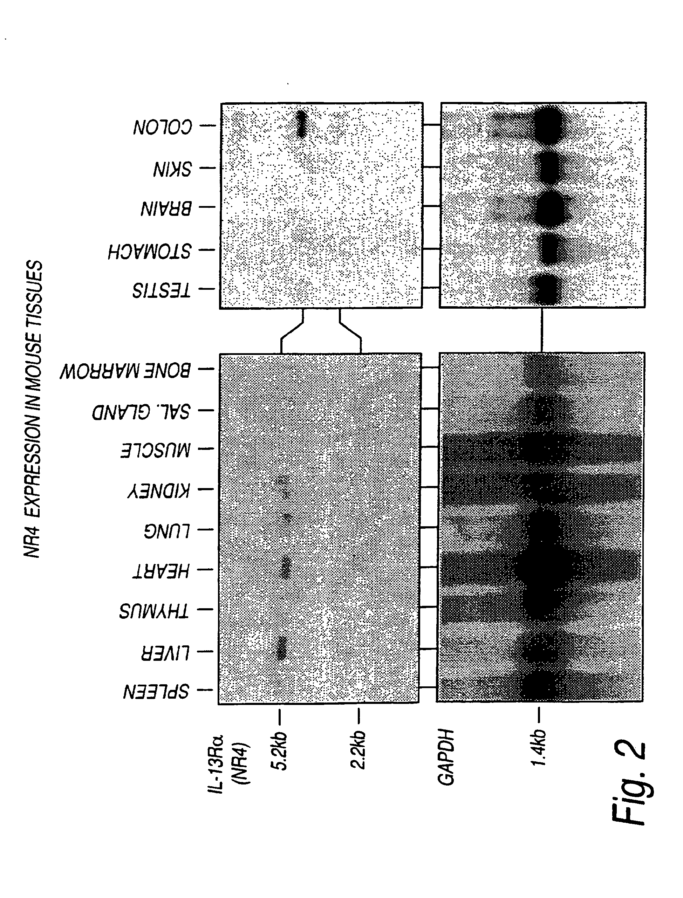

**Figure 2** is a photographic representation showing northern analysis of murine NR4 mRNA expression in selected tissues and organs.

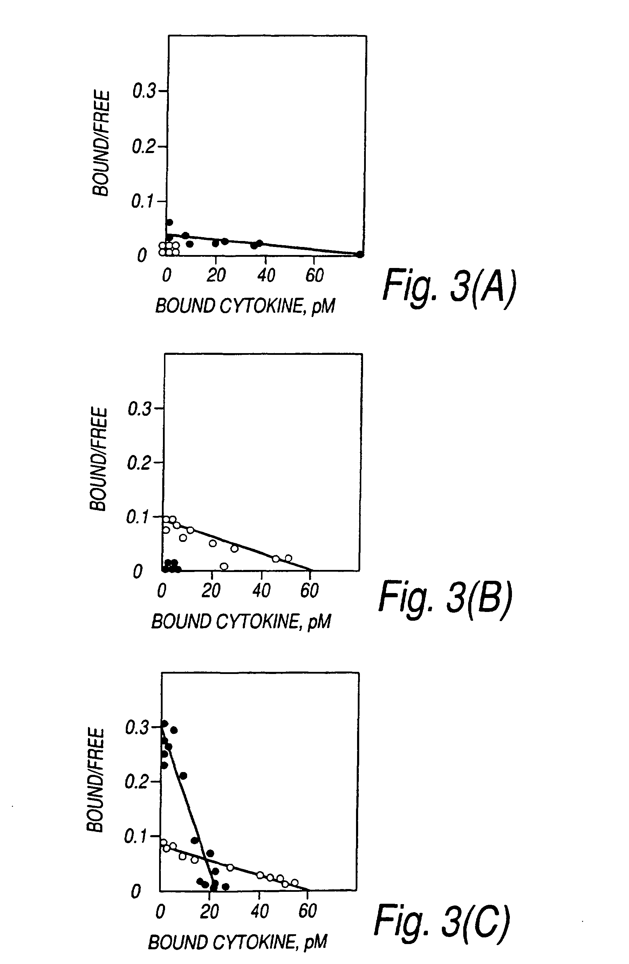

**Figure 3** is a graphical representation depicting saturation isotherms of ¹²⁵I-IL-13 and ¹²⁵I-IL-4 binding; saturation isotherms depicted as Scatchard plots of IL-4 (○) and IL-13 (•) binding to (A) COS cells expressing the IL-13Rα (NR4), (B) CTLL cells and (C) CTLL cells expressing the IL-13Rα (NR4). Data have been normalised to 1x10⁴ COS cells and 1x10⁶ CTLL cells and binding was carried out on ice for 2 to 4 hours.

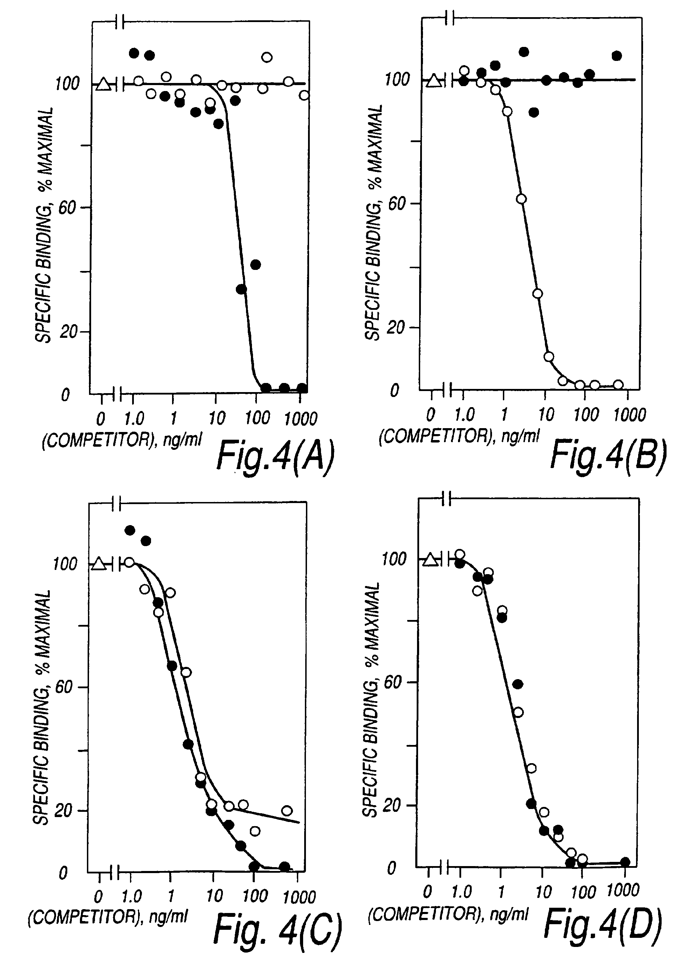

**Figure 4** is a graphical representation showing specificity of IL-4 and IL-13 binding; the ability of IL-4 (○) and IL-13 (•) to compete for ¹²⁵I-IL-13 binding to (A) COS cells expressing the IL-13Rα (NR4) and (C) CTLL cells expressing the IL-13Rα (NR4) or to compete for ¹²⁵I-IL-4 binding to (B) CTLL cells and (D) CTLL cells expressing the IL-13Rα (NR4). Binding was carried out at 4°C for 2 to 4 hours and the data expressed as a percentage of the specific binding observed in the absence of a competitor (Δ).

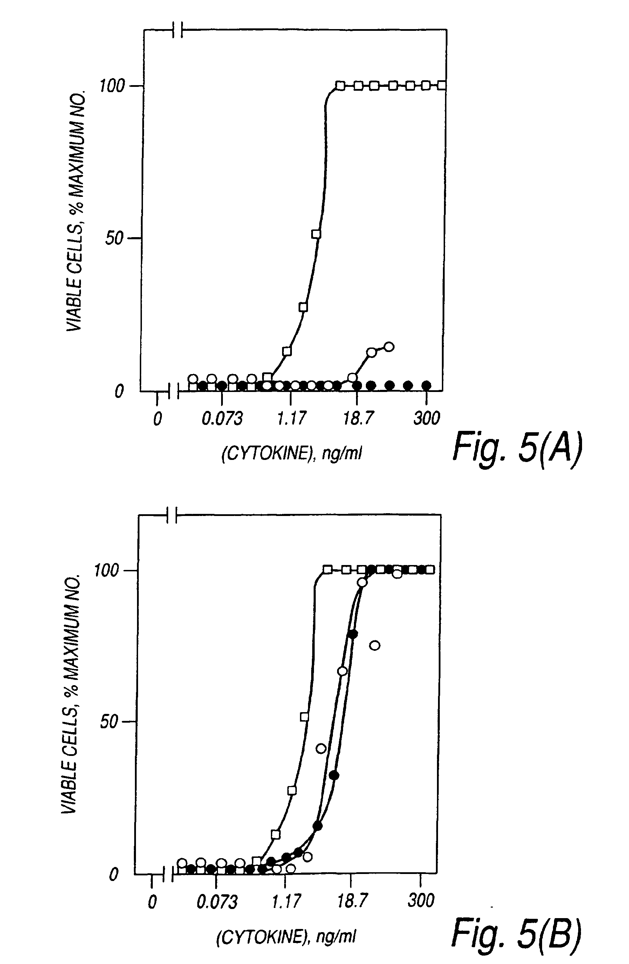

**Figure 5** is a graphical representation showing factor dependent proliferation of cells expressing NR4. Two hundred (A) CTLL cells or (B) CTLL cells expressing the IL-13Rα (NR4) were incubated in the absence of cytokine or with various concentrations of IL-2 (□), IL-4 (○) or IL-13 (•). After 48 hours viable cells were counted and data were expressed as a percentage of the number of viable cells observed with a maximal concentration of IL-2.

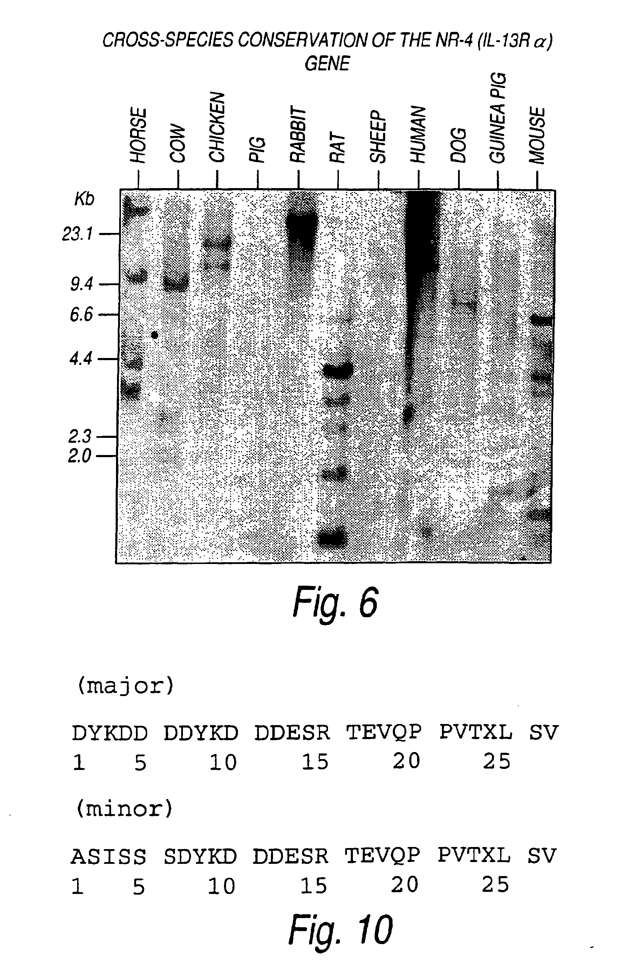

**Figure 6** is a photographic representation showing cross-species conservation of NR4 (IL-13Rα) gene.

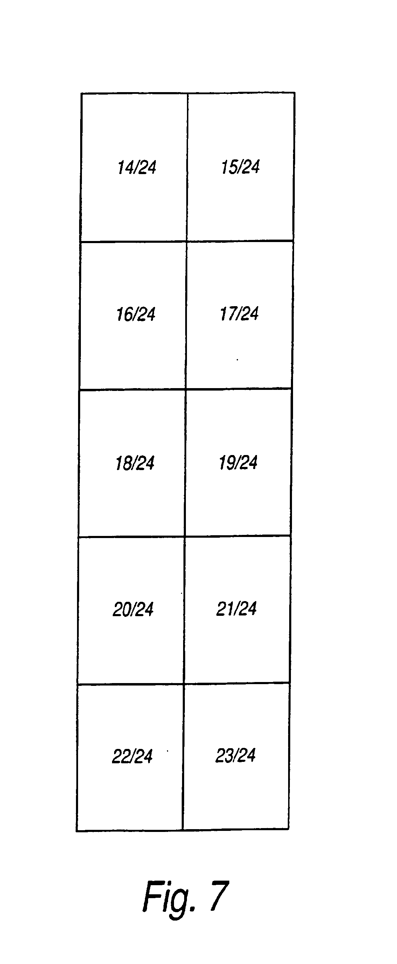

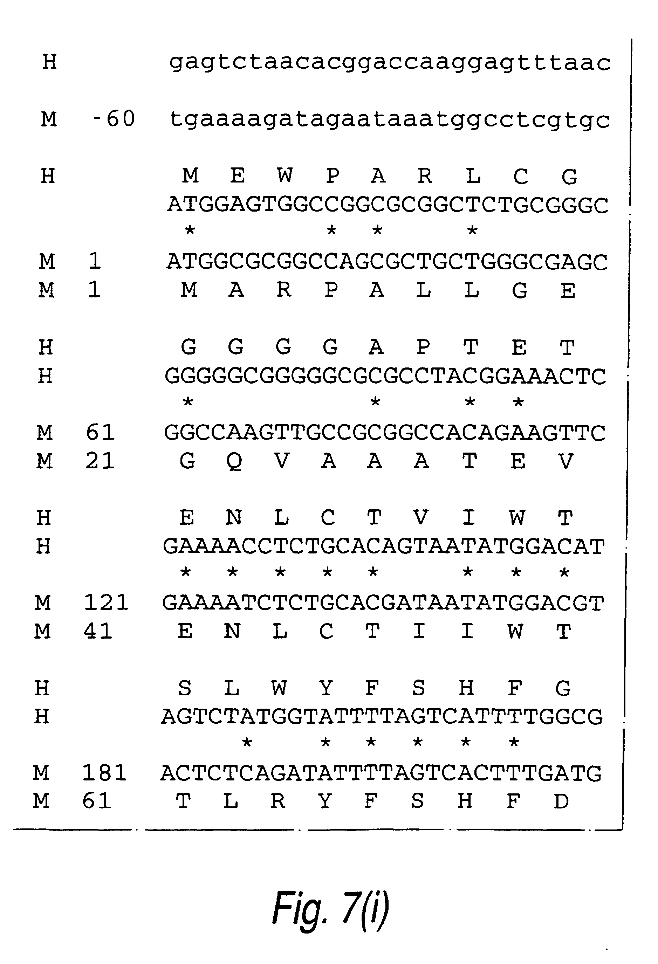

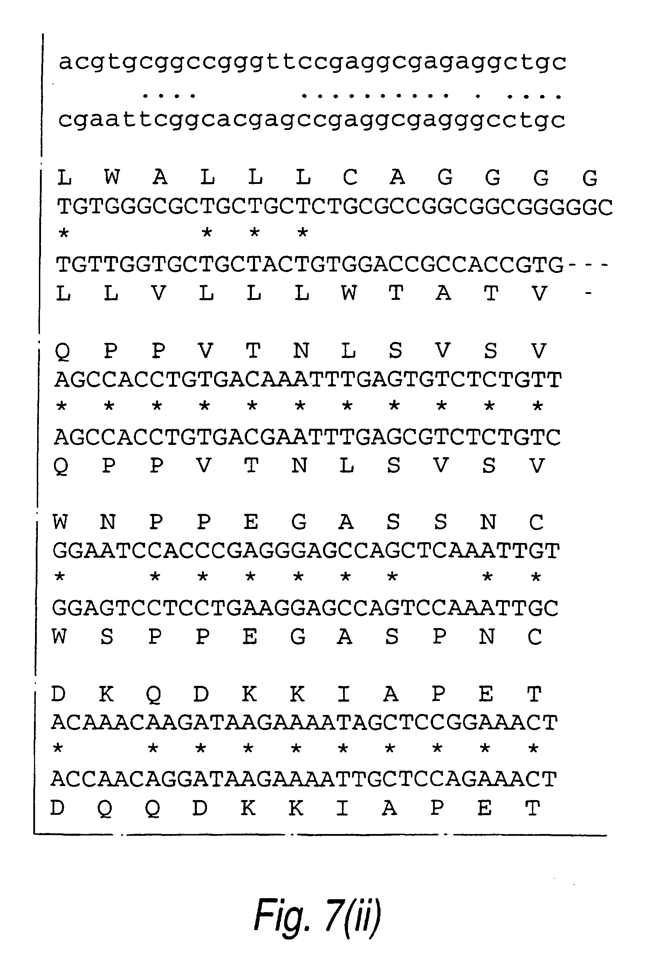

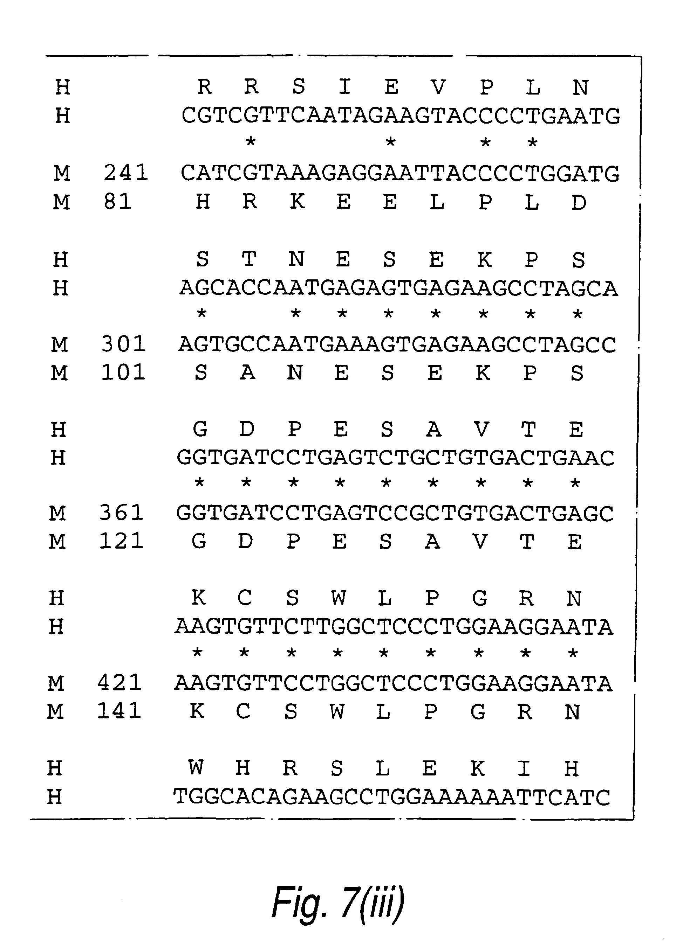

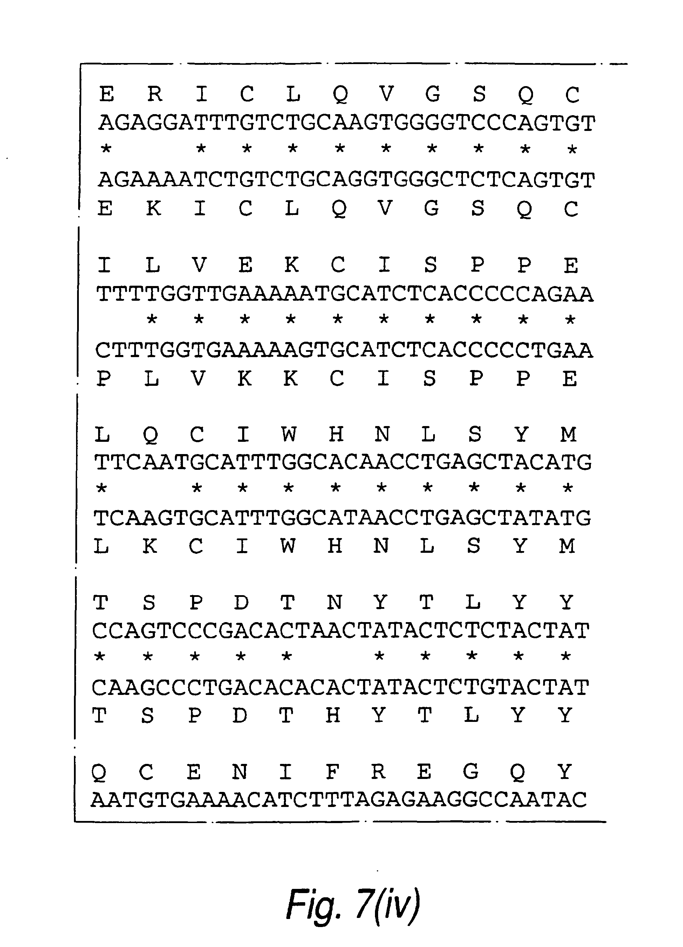

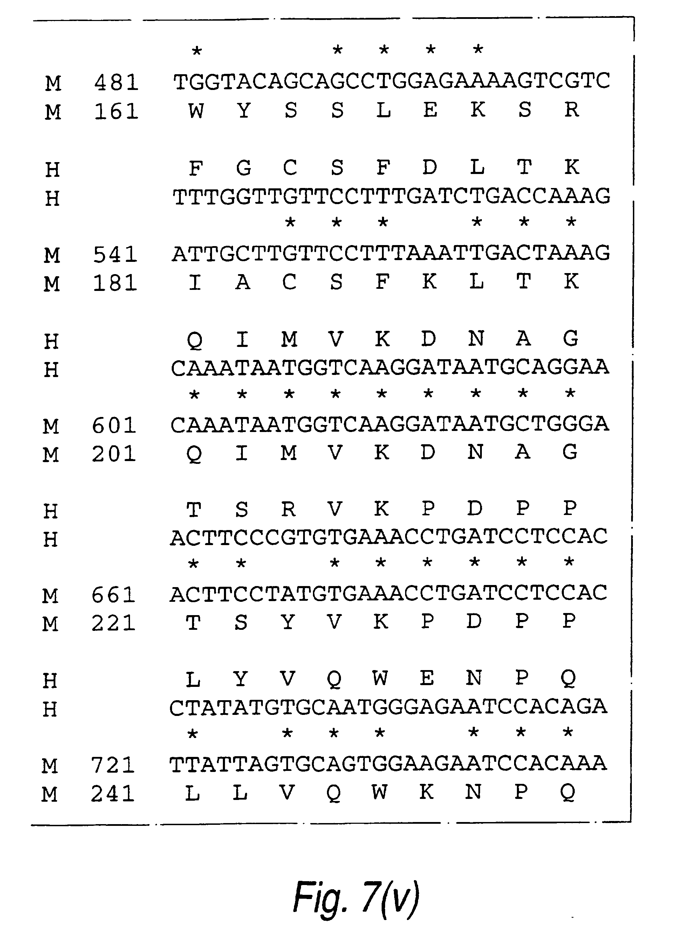

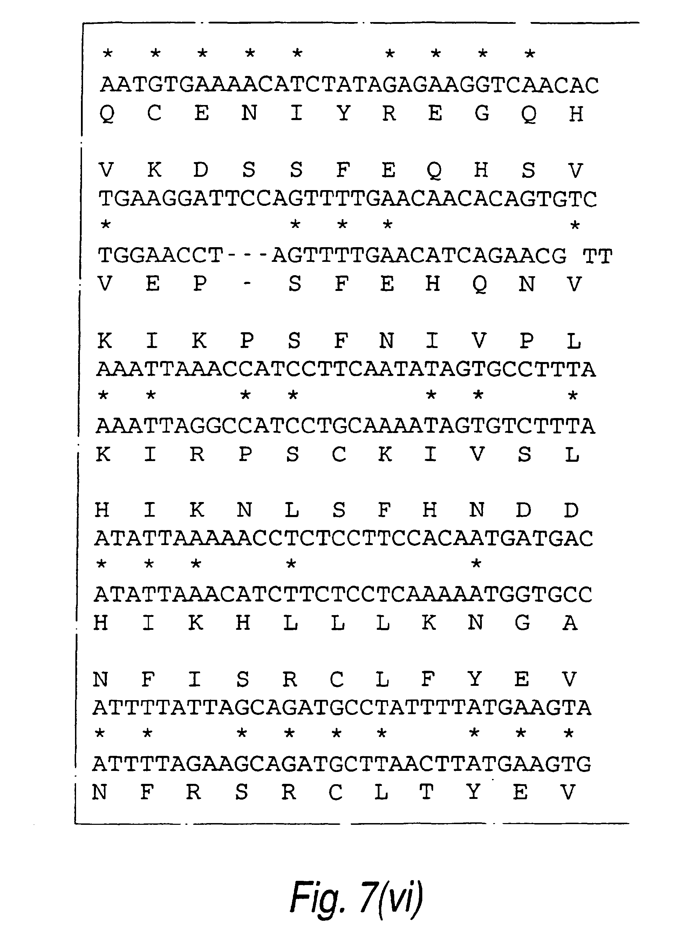

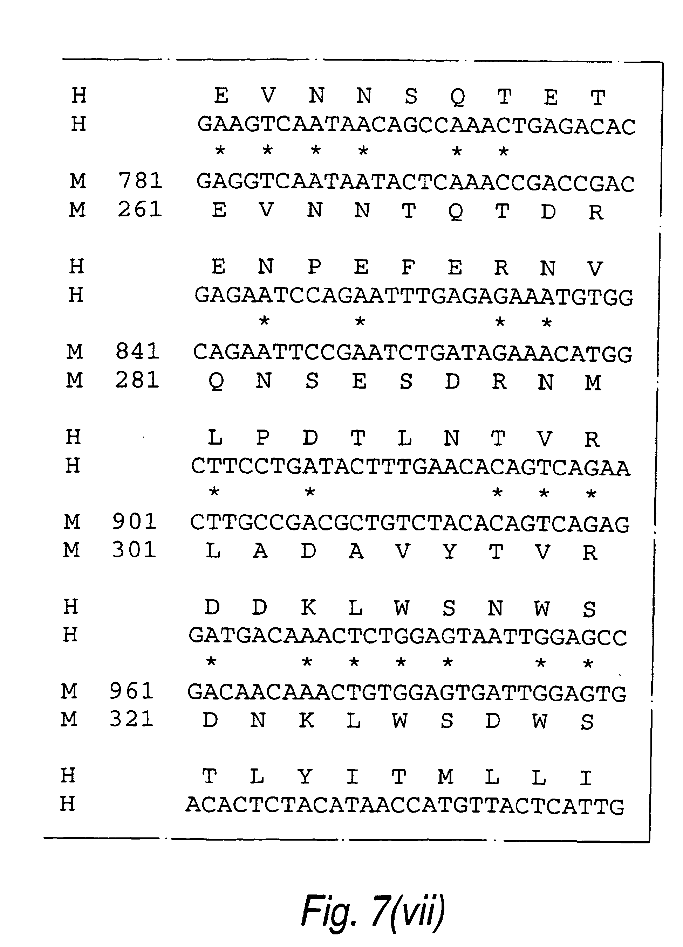

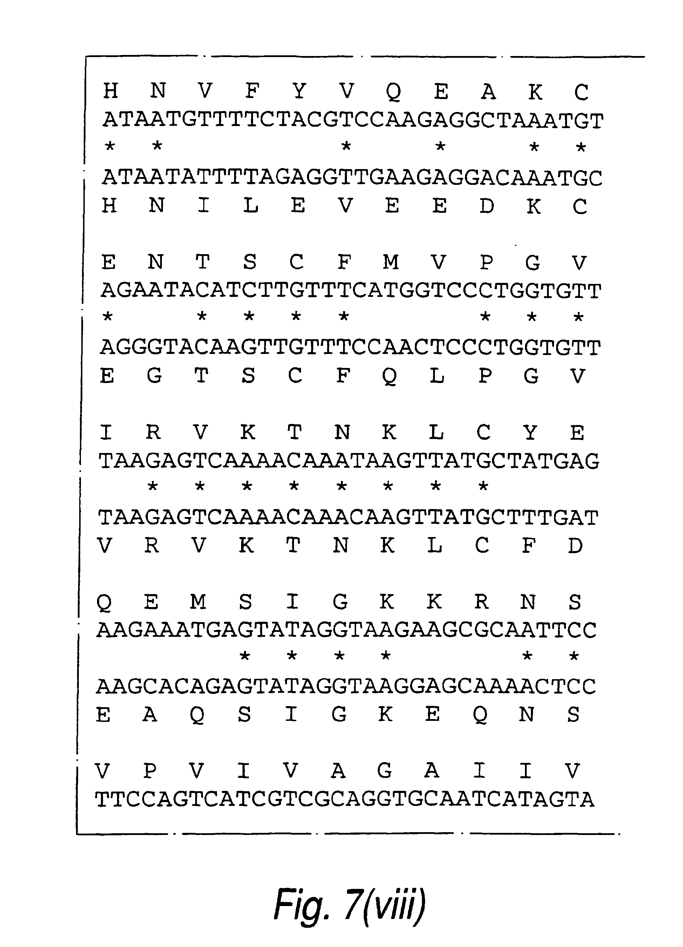

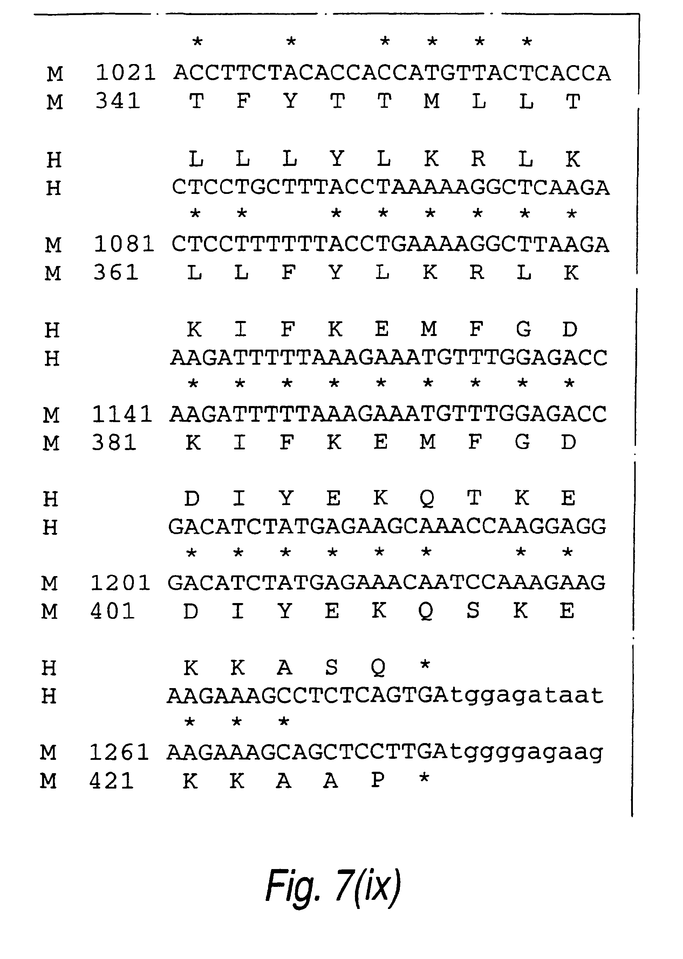

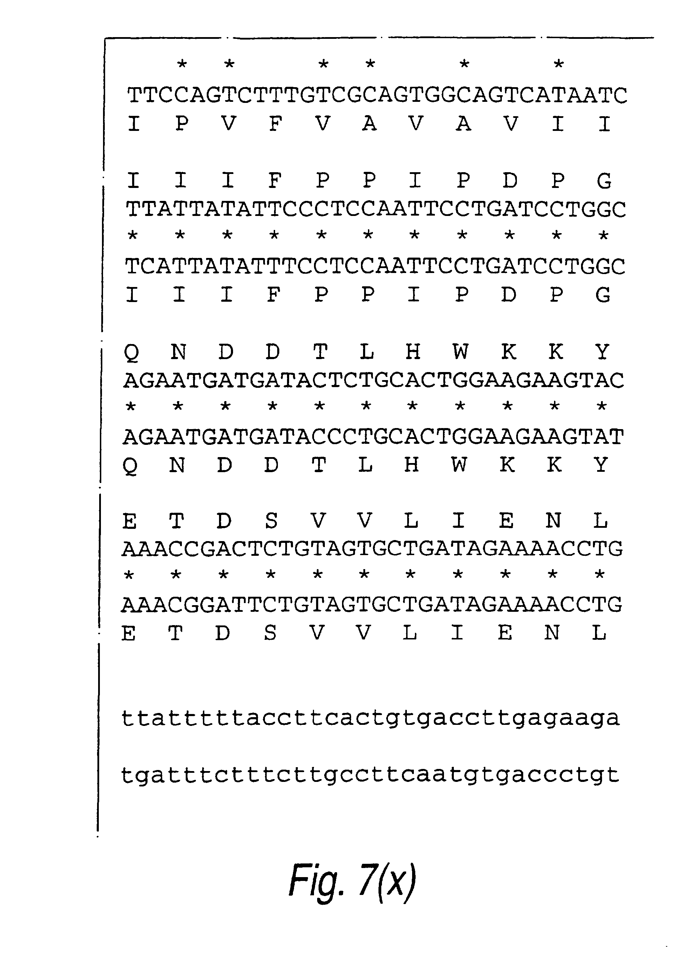

**Figure 7** is a representation of the nucleotide and corresponding amino acid sequence of murine and human NR4 (IL-13Rα) genes. The nucleotide and predicted amino acid sequence of human (H) and murine (M) LL-13Rα (NR4) were aligned by eye, with gaps (-) inserted to optimise the alignment The numbering is for the murine clone, nucleotides that form part of the coding region are shown in upper case, whilst those of the untranslated regions are shown in lower case. Amino acids identical between the predicted murine and human proteins are indicated by (*). DNA encoding the murine signal sequence is underlined, with A26 or T27 being the predicted first amino acid of the mature protein.

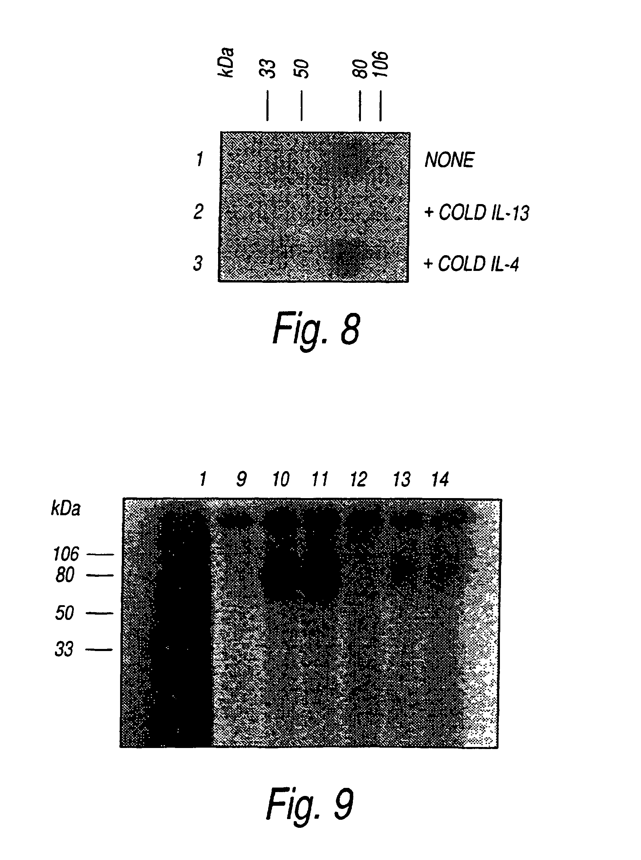

**Figure 8** is a photographic representation showing ¹²⁵I-IL-13 cross-linking to soluble NR4. Lane: ¹²⁵I-IL-13 (100,000 cpm) + 2µg/ml soluble NR4; Lane 2: ¹²⁵I-IL-13 (100,000 cpm) + 2µg/ml soluble NR4 in the presence of excess unlabelled IL-13; Lane 3: ¹²⁵I-IL-13 (100,000 cpm) + 2µg/ml soluble NR4 in the presence of excess unlabelled IL-4.
**Figure 9** is a photographic representation of immunoprecipitation by anti-NR4 polyclonal antisera of cross-linked ¹²⁵I-IL-13 with IL-13Rα (NR4). Lanes 9-11: soluble IL-13Rα (30 µl of 3 µg/ml) cross-linked to ¹²⁵I-IL-13 (750,000 cpm) and immunoprecipitated with control rabbit serum, or with anti-NR4 polyclonal antiserum in the presence or absence of 100 µg/ml FLAG peptide, respectively; Lanes 12-14: soluble IL-13Rα (NR4) (30 µl of 3 µg/ml) cross-linked to ¹²⁵I-IL-13 (750,000 cpm) in the presence of 0.5 µg/ml unlabelled IL-13 and immunoprecipitated with an anti-IL-13Rα (NR4) polyclonal antiserum also in the presence or absence of 100 µg/ml FLAG peptide, respectively.
**Figure 10** is a representation of the N-terminal amino acid sequence of murine NR4. [SEQ ID NOs:10 and 11]

The following single and three letter abbreviations for amino acid residues are used in the specification:

| Amino Acid | Three-letter Abbreviation | One-letter Symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

### EXAMPLE 1

### Isolation of genomic and cDNAs encoding NR4

*Apo*I digested genomic DNA, extracted from an embryonal stem cell line, was cloned into the λZAPII bacteriophage (Stratagene, LaJolla, CA). Approximately 10⁶ plaques from this library were screened with a ³²P-labelled oligonucleotide corresponding to the sequence Trp-Ser-Asp-Trp-Ser (16). Positively hybridising clones were sequenced using an automated DNA sequencer according to the manufacturers instructions (Applied Biosystems, Foster City, CA). One clone appeared to encode for part of a new member of the haemopoietin receptor family. Oligonucleotides were designed on the basis of this genomic DNA sequence and were used in the conventional manner to isolate clones from mouse peritoneal macrophage (Clontech Laboratories, Palo Alto, CA), mouse skin, mouse lung, mouse kidney, and WEHI-3B (Stratagene, LaJolla, CA) λ-bacteriophage cDNA libraries.

### EXAMPLE 2

### Construction of expression vectors and transfection of cells

Using PCR, a derivative of the NR4 cDNA was generated which encoded for the IL-3 signal sequence [SEQ ID NO:5] and an N-terminal FLAG epitope-tag [SEQ ID NO:6] preceding the mature coding region of NR4 (Thr27 to Pro424; Figure 1). The PCR product was cloned into the mammalian expression vector pEF-BOS (17). Constructs were sequenced in their entirety prior to use. Cells were transfected and selected as previously described (16, 18).

### EXAMPLE 3

### Northern blots

Northern blots were performed as previously described (16). The source of hybridisation probes was as follows: NR4 - a PCR product from nucleotide 32 to 984 (Figure 1) and GAPDH - a cDNA fragment spanning nucleotides (19).

### EXAMPLE 4

### Cytokines and experiments using radioiodinated cytokines

IL-2, IL-4, IL-7, IL-9, IL-13 and IL-15 were obtained commercially (R & D Systems, Minneapolis MN). For radioiodination, cytokines were dissolved at a concentration of 100 µg/ml in 10 mM sodium phosphate, 150 mM NaCl (PBS), 0.02% v/v Tween 20 and 0.02% w/v sodium azide at pH 7.4. An amount of 2µg of IL-13 was radioiodinated using the iodine monochloride method (20, 21), while 2µg of IL-4 was radiolabelled using di-iodo-Bolton-Hunter reagent (16). Binding studies and determination of the specific radioactivity and bindability of labelled cytokines were performed as previously described (2).

For cross-linking experiments, recombinant murine IL-13 was produced as a FLAG-tagged protein in *Pichia pastoris*.

For cross-linking assays, aliquots of purified soluble IL-13Rα (NR4) were incubated with ¹²⁵I-IL-13 in the presence or absence of a competitor in a final volume of 20 µl for at least 30 min at 40°C Then 5 µl of a 12 mM solution of BS³ (Bis (Sulfosuccimidyl) suberate) in PBS containing 0.02% v/v Tween-20 was added and the mixtures were incubated for 30 min at 4°C. Samples were mixed with 8 µl of 4XSDS sample buffer and analysed by 13% w/v SDS-PAGE under non-reducing conditions. Gels were dried and visualised by either autoradiography or with a PhosphoImager.

### EXAMPLE 5

### Proliferation Assays

The proliferation of Ba/F3 and CTLL cells in response to cytokines was measured in Lux 60 microwell HL-A plates (Nunc Inc. IL, USA). Cells were washed three times in DMEM containing 20% v/v new born calf serum and resuspended at a concentration of 2 x 10⁴ cells per ml in the same medium. Aliquots of 10µl of the cell suspension were placed in the culture wells with 5µl of various concentrations of purified recombinant cytokines. After 2 days of incubation at 37°C in a fully humidified incubator containing 10% v/v CO₂ in air, viable cells were counted using an inverted microscope.

### EXAMPLE 6

### Cloning and Characterisation of Murine NR4

A library was constructed in λZAP II using *Apo*I digested genomic DNA from embryonal stem cells and screened with a pool of ³²P-labelled oligonucleotides encoding the amino acid sequence Trp-Ser-Asp-Trp-Ser found in many members of the haemopoietin receptor family. One hybridising bacteriophage clone was found to contain a sequence that appeared to encode part of a novel member of the haemopoietin receptor family. This receptor was given the operational name NR4. The sequence of the genomic clone was used to isolate cDNAs encoding NR4 from WEHI-3B cell, peritoneal macrophage, bone marrow, skin and kidney libraries. A composite of the nucleotide sequence [SEQ ID NO:1] and predicted amino acid sequence [SEQ ID NO:2] of these cDNAs is shown in Figure 1. The NR4 cDNA is predicted to encode for a protein of 424 amino acid residues, containing a putative signal sequence and transmembrane domain. The extracellular region of the protein contained an immunoglobulin-like domain (amino acids 27-117), in addition to a typical haemopoietin receptor domain (amino acids 118-340) which includes four conserved cysteine residues and the characteristic Trp-Ser-Asp-Trp-Ser motif [SEQ ID NO:12](Figure 1; in bold as WSXWS). The cytoplasmic tail of the new receptor was 60 amino acids in length.

### EXAMPLE 7

### Expression pattern of NR4 cDNA

The pattern of NR4 mRNA expression was examined by Northern analyses. Two hybridising species of 5.2 and 2.2 kb in length were detected in mRNA from most tissues (Figure 2). NR4 mRNA was not detectable in skeletal muscle (Figure 2). Figure 8 shows expression of NR4 in mouse tissues.

### EXAMPLE 8

### NR4 encodes the IL-13 receptor α-chain (IL-13Rα) - a specific binding subunit of the IL-13 receptor

The apparent molecular mass is from about 50,000 to about 70,000 daltons and more particularly about 55,000 to about 65,000 daltons for NR4 expressed in COS cells estimated from Western blots using an anti-FLAG antibody. This suggested that NR4 might encode the binding subunit of the IL-13 receptor in order to test this possibility, NR4 was expressed in COS cells. Untransfected COS cells expressed relatively low levels of IL-4 and IL-13 receptors Upon transfection with a plasmid containing the NR4 cDNA, the number of IL-13 receptors but not IL-4 receptors expressed by COS cells was dramatically increased (Figure 3A; 100,000 to 500,000 receptors per cell). The affinity of IL-13 for NR4 expressed by COS cells was low (K_{D}-2-10 nM) and binding was specific since it could compete with unlabelled IL-13 (Figure 4A) but not other cytokines including IL-2, IL-4, IL-7, IL-9 or IL-15. These results suggest that NR4 is the IL-13 receptor α-chain (IL-13Rα).

### EXAMPLE 9

### The IL-13Rα (NR4) and the IL-4Rα are shared components of the IL-4 and IL-13 receptors

In order to investigate the relationship between IL-4 and IL-13 receptors, the IL-4 responsive cell line CTLL was examined. Parental CTLL cells expressed a single class of IL-4 receptor (K_{D}∼660 pM; ∼3600 receptors per cell) but no detectable IL-13 receptors (Figure 3B). The IL-4 receptors expressed by CTLL cells appeared to be specific since binding of ¹²⁵I-IL-4 could compete with unlabelled IL-4 but not IL-13 (Figure 4B). Upon expression of the IL-13Rα (NR4) in CTLL cells no change was observed in the number or affinity of IL-4 receptors, while a single class of high affinity IL-13 receptors was detected (Figure 3C; K_{D}∼75 pM; 1350 receptors per cell). The affinity of IL-13 for the IL-13Rα (NR4) expressed in CTLL cells was higher than in COS cells, suggesting that the former expressed a protein capable of interacting with the IL-13Rα (NR4) to increase the affinity for IL-13. A likely candidate based on previous studies is the IL-4Rα. In order to explore this possibility the ability of IL-4 to compete with ¹²⁵I-IL-13 for binding to CTLL cells expressing the IL-13Rα (NR4) was assessed. Figure 4B shows that IL-4 and IL-13 were equally effective in competing for ¹²⁵I-IL-13 binding (IC₅₀ ∼ 300pM; Figure 4C) and, in addition, were able to compete with ¹²⁵I-IL-4 for binding (IC₅₀ ∼ 300 pm; Figure 4D).

### EXAMPLE 10

### Expression of the IL-13Rα (NR4) is necessary for transduction of a proliferative signal by IL-13

CTLL cells require the addition of exogenous cytokines for survival and proliferation. IL-2 was found to be a potent proliferative stimulus for CTLL cells (EC₅₀ ∼ 100-200 pM), while IL-4 was relatively weak (EC₅₀ 2-7 nM) and IL-13 was inactive (Figure 5A). Expression of the IL-13Rα (NR4) in CTLL cells resulted in the ability to survive and proliferate weakly in response to IL-13 (EC₅₀ ∼ 700 pM) and to proliferate somewhat more strongly than parental cells in response to IL-4 (EC₅₀ ∼ 700 pM; Figure 5B).

### EXAMPLE 11

### Cloning of Human IL-13Rα (NR4)

In order to determine whether genes homologous to murine IL-13Rα (NR4) exist in other vertebrate species, a probe encompassing nucleotides 840 to 1270 of murine IL-13Rα (NR4) was hybridised to EcoRI digested genomic DNA from various species. Hybridisation was carried out in 500 mM Na₂HPO₄ (∼5xSSC) at 50°C overnight. The filter was washed in 40 mM Na₂HPO₄ (∼0.2xSSC) at 50°C for 2 hours and exposed to autoradiographic film for 48 hours. Figure 6 illustrates that relatively few (1 to 5) hybridising bands are observed in genomic DNA from various species, including human. This suggests that it is feasible to clone human IL-13Rα (NR4) using a murine cDNA probe. A human bone marrow cDNA library clones in the λZAPII bacteriophage was therefore screened with two probes (nucleotides 82-840 and 840 to 1270) from the murine IL-13Rα (NR4) cDNA. Hybridisation was carried out overnight in 6xSSC, 0.1% w/v SDS at 42°C. Filters were washed at 2xSSC, 0.1% w/v SDS at 50°C for 2 hours and exposed for 48 hours to autoradiographic film. Plaques that hybridised to both murine IL-13Rα (NR4) probes were picked and purified in the conventional manner. The cDNA inserts form the hybridising bacteriophage were excised into the pBluescript plasmid and sequenced in their entirety using an ABI automated sequencer. Figure 7 shows a composite of the sequence of the clones isolated and reveals that the clones encode a protein that shares a high degree of sequence similarity with murine IL-13Rα (NR4). The clones encode the entire coding region of the protein. The high degree of sequence similarity (320/425 amino acids - 75%) predicates that this cDNA is the human homologue of the murine IL-13Rα (NR4). The nucleotide sequence is represented as SEQ ID NO:3 and the amino acid sequence is SEQ ID NO:4.

### EXAMPLE 12

### Soluble Murine IL-13Rα (NR4) binds IL-13

Constructs were engineered to express soluble versions of NR4 with an N-terminal "FLAG" epitope (International Biotechnologies/Eastman Kodak, New Haven CT). First, a derivative of the mammalian expression vector pEF-BOS was generated so that it contained DNA encoding the signal sequence of murine IL-3 (MVLASSTTSIHTMLLLLLMLFHLGLQASIS [SEQ ID NO:5]) and the FLAG epitope (DYKDDDDK [SEQ ID NO:6]), followed by a unique XbaI cloning site. This vector was named pEF/IL3SIG/FLAG. The mature extracellular part of the NR4 coding region (Thr27 to Thr344) was generated by PCR using primers 1478 and 1480. The resulting product was digested with XbaI and was cloned into the XbaI site of pEF/IL3SIG/FLAG to give pEF/IL3SIG/FLAG/sol NR4. The identity of the construct was confirmed by dideoxy sequencing.
OLIGO 1478 5' AGCTTCTAGAACAGAAGTTCAGCCACCTGTG 3' [SEQ ID NO:7]; OLIGO 1480 5' AACTCCACCTTCTACACCACCTGATCTAGA 3' [SEQ ID NO:8].

After transfection into CHO cells, expressed, soluble NR4 was purified from CHO cell-conditioned medium on an anti-FLAG antibody (M2) affinity column by elution with free FLAG peptide (Science Imaging Systems).

Consistent with the low affinity of IL-13 for NR4 expressed by COS cells, purified soluble NR4 appeared unable to bind IL-13 as assessed by gel filtration chromatography. However, using sensitive cross-linking assays, the ability of soluble IL-13Rα (NR4) to bind IL-13 was demonstrated (Figure 8, lane 1). This interaction was competed for by unlabelled IL-13 but not by unlabelled IL-4 (Figure 8, lanes 2 and 3).

### EXAMPLE 13

### A Polyclonal Antisera to Soluble IL-13Rα (NR4)

A polyclonal antiserum to NR4 was prepared by injecting purified soluble NR4 into rabbits which were bled after 3 months. This antisera immunoprecipitated the cross-linked product of ¹²⁵I-IL-13 with soluble NR4 (Figure 9, lane 11) while no immunoprecipitation was observed with pre-immune serum (Figure 9, lane 9). Immunoprecipitation of the complex was not inhibited by the FLAG peptide (Figure 9, lane 10).

The immunoprecipitation assay was conducted as follows:

The cross-linking reactions were terminated by the addition of Tris-HCl, pH 7.5, to a final concentration of 40 mM. The samples were then mixed with 1:50 diluted control rabbit serum or anti-NR4 serum which had been pre-incubated with or without FLAG peptide. After incubation for 30 min at 4°C, the mixtures were added to 40 µl of 50% v/v protein G-Sepharose gel slurry (Pharmacia) and incubated for 30 min at 4°C. The samples were centrifuged and the protein G beads were washed 3 x 0.5 ml PBS, mixed with 40 µl of 2X concentrated SDS-PAGE sample buffer and heated for 2 min at 95°C. The supernatants were analysed by 13 % w/v SDS-PAGE under non-reducing conditions.

### EXAMPLE 14

### N-terminal Amino Acid Sequence of NR4

The N-terminal amino acid sequence of NR4 was determined and is shown in Figure 10.

### EXAMPLE 15

### Assay for IL-13

IL-13 is a cytokine that is implicated in the production of IgE, the immunoglobulin isotype important in allergic diseases such as asthma. Monitoring IL-13 levels may, therefore, be an important diagnostic. Since IL-4 and IL-13 share many biological effects, generating an assay that discriminates these cytokines is also important.

NR4 expressed in COS cells binds ¹²⁵I-IL-13. This binding is inhibited in a dose dependent manner by unlabelled IL-13, in the presence of a large amount of irrelevant protein such as calf serum or human serum. IL-4 shows no ability to compete for ¹²⁵I-IL-13 binding in this situation and, therefore, this assay appears to be specific for IL-13.

The assay is set up by coating soluble NR4 on ELISA plates and using, for example, fluorescent labelled IL-13 as the probe. The presence of unlabelled IL-13 in a test sample then registers as a decrease in the fluorescent signal.

Similar assays are set up that measure both IL-4 and IL-13 by using cells that express NR4 and IL-4 receptor α-chain. These include CTLL cells which normally express IL-4 receptor α-chain and which are engineered to express NR4. Binding of ¹²⁵I-IL-13 or ¹²⁵I-IL-4 can be inhibited by unlabelled forms of both IL-4 and IL-13.

### EXAMPLE 16

### Modifications to IL-4 and IL-13

Mutations are introduced into regions of the molecules that are predicated to be functionally important. In the case of NR4, this includes the region that interacts with IL-13, the region which interacts with IL-4 receptor α-chain or the region that interacts with IL-4 when this cytokine is bound to the IL-4 receptor α-chain. These regions are determined by direct experiment, for example, by solving the structure of NR4 or complexes of NR4 with other proteins like IL-4, IL-13 and the IL-4 receptor α-chain or by modeling these proteins on similar proteins for which structural information exists, for example, the growth hormone/growth hormone receptor complex. Resulting NR4 mutants are then individually tested for improved fuction.

In an alternative method, random mutations are generated in the molecules. Suitable techniques include synthesis of NR4 cDNA using a polymerase and reaction conditions that promote incorporation of the incorrect dNTP and use of a technique called DNA shuffling (23, 24, 25, 26).

After generating random mutants of the cDNA of interest, potentially useful mutants are selected. In the case of NR4, an assay is based on knowledge that if NR4 is expressed in cells which lack IL-4 receptor α-chain (e.g. COS cells), then cells are obtained that cannot bind IL-4 with any detectable affinity and binds IL-13 with low affinity. Thus, if COS cells are transfected with Nr4 and allowed to bind FITC-conjugated IL-4 and phycoerythrin-conjugated IL-13, the unbound ligand washed away, the no IL-4 will bind and any IL-13 that had bound would dissociate during the washing.

If these cells are FACS-sorted, then little or no signal in either the FITC or PE channel would be obtained. COS cells are transfected with 10⁶ to 10⁷ random mutants of NR4 and processed for binding. Any cells sorted which bind the cytokines better than those transfected with wild type NR4 can be FACS sorted. The plasmids containing these "improved" NR4 cDNAs may be recovered, expanded in *E. coli* and used again in COS cells to confirm the improvement. Any mutants that are consistently better can then be used for the introduction of further random changes into an order to get even better molecules. This iterative process may be repeated several times.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

1. Du, X.X. and Williams, D.A. (1994) Blood 83: 2023-2030.
2. Yang, Y.C. and Yin, T. (1992) Biofactors 4: 15-21.
3. Paul, S.R., Bennett, F., Calvetti, J.A., Kelleher, K., Wood, C.R., O'Hara, R.J.J., Leary, A.C., Sibley, B., Clark, S.C., Williams, D.A. and Yang, Y.-C. (1990) Proc. Natl. Acad. Sci. USA 87: 7512.
4. Musashi, M., Clark, S.C., Sudo, T., Urdal, D.L., and Ogawa, M. (1991) Blood 78: 1448-1451.
5. Schibler, K.R., Yang, Y.C. and Christensen, R.D. (1992) Blood 80: 900-3.
6. Tsuji, K., Lyman, S.D., Sudo, T., Clark, S.C., and Ogawa, M. (1992) Blood 79: 2855-60.
7. Burstein, S.A., Mei, R.L., Henthorn, J., Friese, P. and turner, K. (1992) J. Cell. Physiol. 153: 305-12.
8. Hangoc, G., Yin, T., Cooper, S., Schendel, P., Yang, Y.C. and Broxmeyer, H.E. (1993) Blood 81: 965-72.
9. Teramura, M., Kobayashi, S., Hoshino, S., Oshimi, K. and Mizoguchi, H. (1992) Blood 79: 327-31.
10. Yonemura, Y., Kawakita, M., Masuda, T., Fujimoto, K., Kato, K. and Takatsuki, K. (1992) Exp. Hematol. 20: 1011-6.
11. Baumann, H. and Schendel, P. (1991) J. Biol. Chem. 266: 20424-7.
12. Kawashima, I., Ohsumi, J., Mita-Honjo, K., Shimoda-Takano, K., Ishikawa, H., Sakakibara, S., Miyadai, K. and Takiguchi, Y. (1991) Febs. Lett. 283: 199-202.
13. Keller, D.C., Du, X.X., Srour, E.f., Hoffman, R. and Williams, D.A. (1993) Blood 82: 1428-35.
14. McKenzie, A.N.J. and Zurawski, G. (1994) Guidebood to cytokines and their receptors, Oxford University Press. Oxford.
15. Zurawski, G. and de Vries, J.E. (1994) Immunol. Today 15: 19-26.
16. Hilton, D.J., Hilton, A.A., Raicevic, A., Rakar, S., Harrison-Smith, M., Gough, N.M., Begley, C.G., Metcalf, D., Nicola, N.A. and Wilson, T.A. (1994) EMBO J. 13: 4765-4775.
17. Mizushima, S. and Nagata, S. (1990) Nucleic Acids Res. 18: 5322.
18. Lock, P., Metcalf, D. and Nicola, N.A. (1994) Proc. Natl. Acad. Sci. USA 91: 252-256.
19. Dugaiczyk, A. et al (1983) Biochemistry 22: 1605-1613.
20. Contreras, M.A., Bale, W.F. and Spar, I.L. (1983) Methods in Enzymol. 92: 277-292.
21. Hilton, D.J. and Nicola, N.A. (1992) J. Biol. Chem. 267: 10238-10247.
22. Sambrook, J., Fritsch, E.F. and Maniatis, T. (1989) Molecular cloning: A Laboratory Manual Cold Spring Harbor Laboratory, Cold Spring Harbor, New York.
23. Stemmer, W.P.C., (1994) Nature 370: 389-391.
24. Stemmer, W.P.C., (1995) Biotechnology 13: 549-553.
25. Gassmann et al (1995) Proc. Natl. Acad. Sci. USA 92: 1292-1296.
26. Grameri et al (1996) Nature Biotechnology 14: 315-319.

## Claims

1. An isolated nucleic acid molecule comprising a sequence of nucleotides which encodes or is complementary to a sequence which encodes a polypeptide,
said polypeptide having an amino acid sequence
(i) as set forth in SEQ ID NO:2 or SEQ ID NO:4 or a part thereof which comprises the IL-13 binding portion of said amino acid sequence; or
(ii) having at least about 70% similarity to a sequence in (i) ; or said sequence of nucleotides having a nucleotide sequence
(iii) as set forth in SEQ ID NO:1 or SEQ ID NO:3 or a part thereof which comprises the sequence of said nucleotide sequence which encodes the IL-13 binding portion; or
(iv) having at least about 70% similarity to a sequence in (iii), or
(v) capable of hybridising to the nucleotide sequence as set forth in SEQ ID NO:1 or SEQ ID NO:3 or a complementary form thereof under high stringency conditions, using from at least 31% v/v to at least 50% v/v formamide and from at least 0.01M to at least 0.15M salt for hybridisation, and at least 0.01M to at least 0.15M for washing, or
(vi) which is a complementary sequence of a sequence of (iii) to (v),
wherein said polypeptide is capable of interaction with IL-13.

2. An isolated nucleic acid molecule as claimed in claim 1 wherein said sequence of (v) comprises a nucleotide sequence coding for an amino acid sequence having at least 70% similarity to the sequence set forth in SEQ ID NO:2 or SEQ ID NO:4.

3. An isolated nucleic acid molecule according to claim 1 or 2 which encodes a haemopoietin receptor capable of interaction with IL-13, which interaction is capable of competitive inhibition by IL-4 in cells which express an IL-4 receptor α-chain.

4. An isolated nucleic acid molecule as claimed in any one of claims 1 to 3 wherein the encoded polypeptide is in the form of a fusion protein.

5. An isolated nucleic acid molecule as claimed in any one of claims 1 to 3 wherein the encoded polypeptide is in a soluble form.

6. An isolated nucleic acid molecule as claimed in any one of claims 1 to 3 wherein the encoded polypeptide is a mature IL-13 receptor α-chain or a part thereof which comprises the IL-13 binding portion of said amino acid sequence.

7. A genetic construct comprising a nucleic acid molecule according to any one of claims 1 to 6 operably linked to a promoter capable of directing expression of said nucleic acid molecule in a host cell.

8. A recombinant polypeptide comprising a sequence of amino acids as defined in sequences (i) or (ii) in claim 1 or encoded by a nucleotide sequence as defined in sequences (iii) or (iv) in claim 1 or a complementary sequence thereof, said polypeptide being capable of interaction with IL-13.

9. A recombinant polypeptide according to claim 8 wherein the interaction with IL-13 is competitively inhibited by IL-4 in cells which express an IL-4 receptor α-chain.

10. A recombinant polypeptide according to claim 8 wherein the interaction with IL-13 is with low affinity.

11. A recombinant polypeptide according to claim 8 wherein the interaction with IL-13 is with medium to high affinity.

12. A recombinant polypeptide according to any one of claims 8 to 11 wherein said polypeptide has a molecular weight of from about 50,000 to about 70,000 daltons as determined by Western blot analysis when expressed in COS cells.

13. A recombinant polypeptide as claimed in any one of claims 8 to 12, wherein said polypeptide is in the form of a fusion protein.

14. A recombinant polypeptide as claimed in any one of claims 8 to 12 wherein said polypeptide is in a soluble form.

15. A recombinant polypeptide as claimed in any one of claims 8 to 12 wherein said polypeptide is a mature IL-13 receptor α-chain or a part thereof which comprises the IL-13 binding portion of said amino acid sequence.

16. An antibody specific to the recombinant polypeptide according to any one of claims 8 to 15.

17. An antibody according to claim 16 wherein said antibody is a monoclonal antibody.

18. A hybrid haemopoietin receptor capable of interaction with at least two cytokines wherein at least one of said cytokines is IL-13 and wherein said hybrid receptor comprises an amino acid sequence which includes all of the amino acid sequence set forth in SEQ ID NO:2 or SEQ ID NO:4 or a part thereof which comprises the IL-13 binding portion of said amino acid sequence.

19. A hybrid haemopoietin receptor capable of high affinity interaction with at least one cytokine wherein at least one of said cytokines is IL-13 and wherein said hybrid receptor comprises an amino acid sequence which includes all of the amino acid sequence set forth in SEQ ID NO:2 or SEQ ID NO:4 or a part thereof which comprises the IL-13 binding portion of said amino acid sequence.

20. A hybrid haemopoietin receptor according to claim 18 or 19 capable of interaction with IL-4.

21. A hybrid haemopoietin receptor according to claim 19 capable of interaction with a cytokine selected from IL-2, IL-5, IL-7, IL-9 and IL-15.

22. A pharmaceutical composition comprising a recombinant polypeptide according to any one of claims 8 to 15 and one or more pharmaceutically acceptable carriers and/or diluents.

23. A pharmaceutical composition comprising a nucleic acid molecule according to any one of claims 1 to 6 and one or more pharmaceutically acceptable carriers and/or diluents.

24. A recombinant polypeptide as defined in any one of claims 8 to 15 for use as a medicament.

25. A recombinant polypeptide as defined in any one of claims 8 to 15 for treating asthma, allergy or a condition exacerbated by IgE production.

26. Use of a recombinant polypeptide as defined in any one of claims 8 to 15 in the preparation of a medicament for treating asthma, allergy or a condition exacerbated by IgE production.

27. A method of producing a recombinant polypeptide as defined in any one of claims 8 to 15, said method comprising culturing cells comprising the genetic construct according to claim 7 for a time and under conditions sufficient to express the nucleic acid molecule in said genetic construct to produce a recombinant polypeptide and isolating said recombinant polypeptide.

28. Isolated animal cells comprising the genetic construct according to claim 7 and which express the recombinant polypeptide as defined in any one of claims 8 to 15 produced by the method according to claim 27.

29. A fusion protein comprising a first portion capable of interaction with IL-13, wherein said first portion is encoded by a nucleic acid molecule as defined in any one of claims 1 to 6, and a second portion derived from a haemopoietin receptor, a receptor tyrosine kinase, a TNF/NGF receptor or a G protein coupled receptor.

30. A fusion protein according to claim 29 wherein the second portion comprises all or a functional portion of IL-13 binding protein, IL-4 receptor α-chain, IL-2 receptor γ-chain or a receptor for a cytokine implicated in asthma or allergy.

31. An antibody as claimed in claim 16 or 17 for use as a medicament.

32. An antibody as claimed in claim 16 or 17 wherein said antibody inhibits the interaction of a polypeptide according to any one of claims 8 to 15 with the IL-4 receptor α-chain.

## Patentansprüche

1. Isoliertes Nukleinsäuremolekül, umfassend eine Sequenz von Nukleotiden, die codiert für ein Polypeptid oder komplementär ist zu einer Sequenz, die für ein Polypeptid codiert,
wobei das Polypeptid eine Aminosäuresequenz aufweist
(i) wie in SEQ ID NO:2 oder SEQ ID NO:4 oder einem Teil davon, umfassend den IL-13-Bindungsteil der Aminosäuresequenz, angegeben; oder
(ii) mit mindestens etwa 70% Ähnlichkeit mit einer Sequenz in (i); oder worin die Sequenz von Nukleotiden eine Nukleotidsequenz aufweist
(iii) wie in SEQ ID NO:1 oder SEQ ID NO:3 oder einem Teil davon, umfassend die Sequenz der Nukleotidsequenz, die für den IL-13-Bindungsteil codiert, angegeben; oder
(iv) mindestens etwa 70% Ähnlichkeit mit einer Sequenz in (iii); oder
(v) in der Lage ist zum Hybridisieren an die Nukleotidsequenz wie in SEQ ID NO:1 oder SEQ ID NO:3 angegeben, oder eine komplementäre Form davon, unter hochstringenten Bedingungen, unter Verwendung von mindestens 31% V/V bis mindestens 50% V/V Formamid und von mindestens 0,01 M bis mindestens 0,15 M Salz für Hybridisierung und mindestens 0,01 M bis mindestens 0,15 M für Waschen, oder
(vi) die eine komplementäre Sequenz einer Sequenz von (iii) bis (v) ist,
worin das Polypeptid in der Lage zur Wechselwirkung mit IL-13 ist.

2. Isoliertes Nukleinsäuremolekül nach Anspruch 1, worin die Sequenz von (v) eine Nukleotidsequenz umfasst, die für eine Aminosäuresequenz codiert mit mindestens 70% Ähnlichkeit mit der in SEQ ID NO:2 oder SEQ ID NO:4 angegebenen Sequenz hat.

3. Isoliertes Nukleinsäuremolekül nach Anspruch 1 oder 2, das für einen Hämatopoietinrezeptor codiert, der in der Lage ist zur Wechselwirkung mit IL-13, wobei die Wechselwirkung in der Lage ist zum kompetitiven Inhibieren durch IL-4 in Zellen, die eine IL-4-Rezeptor-α-Kette exprimieren.

4. Isoliertes Nukleinsäuremolekül nach einem der Ansprüche 1 bis 3, worin das codierte Polypeptid in der Form eines Fusionsproteins ist.

5. Isoliertes Nukleinsäuremolekül nach einem der Ansprüche 1 bis 3, worin das codierte Polypeptid in löslicher Form ist.

6. Isoliertes Nukleinsäuremolekül nach einem der Ansprüche 1 bis 3, worin das codierte Polypeptid eine reife IL-13-Rezeptor-α-Kette oder ein Teil davon ist, der den IL-13-Bindungsteil der Aminosäuresequenz umfasst.

7. Genetisches Konstrukt, umfassend ein Nukleinsäuremolekül nach einem der Ansprüche 1 bis 6, operativ verbunden mit einem Promotor, der in der Lage ist zum Steuern der Expression des Nukleinsäuremoleküls in einer Wirtszelle.

8. Rekombinantes Polypeptid, umfassend eine Sequenz von Aminosäuren wie in den Sequenzen (i) oder (ii) in Anspruch 1 definiert oder codiert durch eine Nukleotidsequenz wie definiert in den Sequenzen (iii) oder (iv) in Anspruch 1 oder eine komplementäre Sequenz davon, wobei das Polypeptid in der Lage ist zur Wechselwirkung mit IL-13.

9. Rekombinantes Polypeptid nach Anspruch 8, worin die Wechselwirkung mit IL-13 kompetitiv inhibiert wird durch IL-4 in Zellen, die eine IL-4-Rezeptor-α-Kette exprimieren.

10. Rekombinantes Polypeptid nach Anspruch 8, worin die Wechselwirkung mit IL-13 mit geringer Affinität ist.

11. Rekombinantes Polypeptid nach Anspruch 8, worin die Wechselwirkung mit IL-13 mit mittlerer bis hoher Affinität ist.

12. Rekombinantes Polypeptid nach einem der Ansprüche 8 bis 11, worin das Polypeptid gemäß Bestimmung durch Western Blot-Analyse ein Molekulargewicht von etwa 50000 bis etwa 70000 Dalton bei Expression in COS-Zellen aufweist.

13. Rekombinantes Polypeptid nach einem der Ansprüche 8 bis 12, worin das Polypeptid in der Form eines Fusionsproteins ist.

14. Rekombinantes Polypeptid nach einem der Ansprüche 8 bis 12, worin das Polypeptid in einer löslichen Form ist.

15. Rekombinantes Polypeptid nach einem der Ansprüche 8 bis 12, worin das Polypeptid eine reife IL-13-Rezeptor-α-Kette oder ein Teil davon ist, der den IL-13-Bindungsteil der Aminosäuresequenz umfasst.

16. Antikörper, der spezifisch für das rekombinante Polypeptid nach einem der Ansprüche 8 bis 15 ist.

17. Antikörper nach Anspruch 16, worin der Antikörper ein monoklonaler Antikörper ist.

18. Hybridhämatopoietin-Rezeptor, der in der Lage ist zur Wechselwirkung mit mindestens zwei Cytokinen, worin mindestens eines der Cytokine IL-13 ist und worin der Hybridrezeptor eine Aminosäuresequenz umfasst, die das Gesamte der Aminosäuresequenz, die in SEQ ID NO:2 oder SEQ ID NO:4 angegeben ist oder einen davon Teil umfasst, der den IL-13-Bindungsteil der Aminosäuresequenz umfasst.

19. Hybridhämatopoietin-Rezeptor, der in der Lage ist zu Hochaffinitäts-Wechselwirkung mit mindestens einem Cytokin, worin mindestens eines der Cytokine IL-13 ist und worin der Hybridrezeptor eine Aminosäuresequenz umfasst, die das Gesamte der Aminosäuresequenz, die in SEQ ID NO:2 oder SEQ ID NO:4 angegeben ist, oder einen davon Teil umfasst, der den IL-13-Bindungsteil der Aminosäuresequenz umfasst.

20. Hybridhämatopoietin-Rezeptor nach Anspruch 18 oder 19, der in der Lage ist zur Wechselwirkung mit IL-4.

21. Hybridhämatopoietin-Rezeptor nach Anspruch 19, der in der Lage ist zur Wechselwirkung mit einem Cytokin, ausgewählt aus IL-2, IL-5, IL-7, IL-9 und IL-15.

22. Pharmazeutische Zusammensetzung, umfassend ein rekombinantes Polypeptid nach einem der Ansprüche 8 bis 15 und einen oder mehrere pharmazeutisch verträgliche Träger und/oder Verdünnungsmittel.

23. Pharmazeutische Zusammensetzung, umfassend ein Nukleinsäuremolekül nach einem der Ansprüche 1 bis 6 und einen oder mehrere pharmazeutisch verträglich Träger und/oder Verdünnungsmittel.

24. Rekombinantes Polypeptid nach einem der Ansprüche 8 bis 15 zur Verwendung als ein Medikament.

25. Rekombinantes Polypeptid nach einem der Ansprüche 8 bis 15 zur Behandlung von Astma, Allergie oder eines Zustands, der verschlimmert wird durch IGE-Produktion.

26. Verwendung eines rekombinanten Polypeptids nach einem der Ansprüche 8 bis 15 bei der Herstellung eines Medikaments zur Behandlung von Astma, Allergie oder eines Zustands, der verschlimmert wird durch IGE-Produktion.

27. Verfahren zum Produzieren eines rekombinanten Polypeptids nach einem der Ansprüche 8 bis 15, wobei das Verfahren das Kultivieren von Zellen umfasst, die das genetische Konstrukt nach Anspruch 7 umfassen, für eine Zeit und unter Bedingungen, die ausreichend sind zum Exprimieren des Nukleinsäuremoleküls in dem genetischen Konstrukt zum Produzieren eines rekombinanten Polypeptids und Isolieren des rekombinanten Polypeptids.

28. Isolierte Tierzellen, umfassend das genetische Konstrukt nach Anspruch 7, und welche das rekombinante Polypeptid nach einem der Ansprüche 8 bis 15 exprimieren, produziert durch das Verfahren nach Anspruch 27.

29. Fusionsprotein, umfassend einen ersten Teil, der in der Lage ist zur Wechselwirkung mit IL-13, worin der erste Teil codiert wird durch ein Nukleinsäuremolekül nach einem der Ansprüche 1 bis 6, und einen zweiten Teil, der von einem Hämatopoietinrezeptor, einer Rezeptortyrosinkinase, einem TNF/NGF-Rezeptor oder einem G-Protein-gekoppelten Rezeptor stammt.

30. Fusionsprotein nach Anspruch 29, worin der zweite Teil das Gesamte oder einen funktionalen Teil des IL-13-Bindungsproteins der IL-4-Rezeptor-α-Kette, IL-2-Rezeptor-γ-Kette oder eines Rezeptors für ein Cytokin, das bei Astma oder Allergie impliziert ist, umfasst.

31. Antikörper nach Anspruch 16 oder 17 zur Verwendung als ein Medikament.

32. Antikörper nach einem der Ansprüche 16 oder 17, worin der Antikörper die Wechselwirkung eines Polypeptids nach einem der Ansprüche 8 bis 15 mit der IL-4-Rezeptor-α-Kette inhibiert.

## Revendications

1. Molécule d'acide nucléique isolée, comprenant une séquence de nucléotides qui code pour un polypeptide ou qui est complémentaire d'une séquence qui code pour un polypeptide,
ledit polypeptide présentant une séquence d'acides aminés :
(i) telle que représentée par la SEQ ID n° 2 ou par la SEQ ID n° 4, ou par une partie de celles-ci qui comprend la partie de liaison à l'IL-13 de ladite séquence d'acides aminés ; ou
(ii) qui présente au moins environ 70 % de similarité avec une séquence mentionné au point (i); ou
ladite séquence de nucléotides présentant une séquence de nucléotides :
(iii) telle que représentée par la SEQ ID n° 1 ou par la SEQ ID n° 3, ou par une partie de celles-ci qui comprend la séquence de ladite séquence de nucléotides qui code pour la partie de liaison à l'IL-13 ; ou
(iv) qui présente au moins environ 70 % de similarité avec une séquence mentionnée au point (iii), ou
(v) qui est capable de s'hybrider avec la séquence de nucléotides telle que représentée par la SEQ ID n° 1 ou par la SEQ ID n° 3 ou par une forme complémentaire de celles-ci, dans des conditions de stringence élevée, en utilisant au moins 31 % v/v à au moins 50 % v/v de formamide et au moins 0,01 M à au moins 0,15 M de sel pour l'hybridation, et au moins 0,01 M à au moins 0,15 M pour le lavage, ou
(vi) qui présente une séquence complémentaire à une séquence mentionnée aux points (iii) à (v),
ledit polypeptide étant capable d'interagir avec l'IL-13.

2. Molécule d'acide nucléique isolée selon la revendication 1, dans laquelle ladite séquence mentionnée au point (v) comprend une séquence de nucléotides codant pour une séquence d'acides aminés présentant au moins 70 % de similarité avec la séquence représentée par la SEQ ID n° 2 ou par la SEQ ID n° 4.

3. Molécule d'acide nucléique isolée selon la revendication 1 ou 2, qui code pour un récepteur de l'hémopoïétine capable d'interagir avec l'IL-13, l'interaction en question pouvant faire l'objet d'une inhibition compétitive par le biais de l'IL-4 dans des cellules qui expriment une chaîne α du récepteur de l'IL-4.

4. Molécule d'acide nucléique isolée selon l'une quelconque des revendications 1 à 3, dans laquelle le polypeptide codé se présente sous la forme d'une protéine de fusion.

5. Molécule d'acide nucléique isolée selon l'une quelconque des revendications 1 à 3, dans laquelle le polypeptide codé se présente sous une forme soluble.

6. Molécule d'acide nucléique isolée selon l'une quelconque des revendications 1 à 3, dans laquelle le polypeptide codé est une chaîne α mature du récepteur de l'IL-13, ou une partie de celle-ci qui comprend la partie de liaison à l'IL-13 de ladite séquence d'acides aminés.

7. Construction génétique, comprenant une molécule d'acide nucléique selon l'une quelconque des revendications 1 à 6 reliée de manière opérationnelle à un promoteur capable de diriger l'expression de ladite molécule d'acide nucléique dans une cellule hôte.

8. Polypeptide recombinant, comprenant une séquence d'acides aminés telle que définie dans les séquences mentionnées au point (i) ou (ii) dans la revendication 1, ou codé par une séquence de nucléotides telle que définie dans les séquences mentionnées au point (iii) ou (iv) dans la revendication 1 ou une séquence complémentaire de celles-ci, ledit polypeptide étant capable d'interagir avec l'IL-13.

9. Polypeptide recombinant selon la revendication 8, dans lequel l'interaction avec l'IL-13 fait l'objet d'une inhibition compétitive par l'IL-4 dans des cellules qui expriment une chaîne α du récepteur de l'IL-4.

10. Polypeptide recombinant selon la revendication 8, dans lequel l'interaction avec l'IL-13 se fait avec une faible affinité.

11. Polypeptide recombinant selon la revendication 8, dans lequel l'interaction avec l'IL-13 se fait avec une affinité intermédiaire à élevée.

12. Polypeptide recombinant selon l'une quelconque des revendications 8 à 11, dans lequel ledit polypeptide présente un poids moléculaire d'environ 50 000 à environ 70 000 daltons comme déterminé par une analyse de transfert de protéïnes de Western dans le cas d'une expression dans des cellules COS.

13. Polypeptide recombinant selon l'une quelconque des revendications 8 à 12, dans lequel ledit polypeptide se présente sous la forme d'une protéine de fusion.

14. Polypeptide recombinant selon l'une quelconque des revendications 8 à 12, dans lequel ledit polypeptide se présente sous une forme soluble.

15. Polypeptide recombinant selon l'une quelconque des revendications 8 à 12, dans lequel ledit polypeptide est une chaîne α mature du récepteur de l'IL-13,
ou une partie de celle-ci qui comprend la partie de liaison à l'IL-13 de ladite séquence d'acide aminés.

16. Anticorps spécifique au polypeptide recombinant selon l'une quelconque des revendications 8 à 15.

17. Anticorps selon la revendication 16, dans lequel ledit anticorps est un anticorps monoclonal.

18. Récepteur d'hémopoïétine hybride capable d'interagir avec au moins deux cytokines, dans lequel au moins l'une desdites cytokines est l'IL-13, et dans lequel ledit récepteur hybride comprend une séquence d'acides aminés qui comprend l'intégralité de la séquence d'acides aminés représentée par la SEQ ID n° 2 ou par la SEQ ID n° 4 ou par une partie de celles-ci qui comprend la partie de liaison à l'IL-13 de ladite séquence d'acides aminés.

19. Récepteur d'hémopoïétine hybride capable d'interagir avec une affinité élevée avec au moins une cytokine, dans lequel au moins l'une desdites cytokines est l'IL-13, et dans lequel ledit récepteur hybride comprend une séquence d'acides aminés qui comprend l'intégralité de la séquence d'acides aminés représentée par la SEQ ID n° 2 ou par la SEQ ID n° 4 ou par une partie de celles-ci qui comprend la partie de liaison à l'IL-13 de ladite séquence d'acides aminés.

20. Récepteur d'hémopoïétine hybride selon la revendication 18 ou 19, capable d'interagir avec l'IL-4.

21. Récepteur d'hémopoïétine hybride selon la revendication 19, capable d'interagir avec une cytokine choisie parmi l'IL-2, l'IL-5, l'IL-7, l'IL-9 et l'IL-15.

22. Composition pharmaceutique comprenant un polypeptide recombinant selon l'une quelconque des revendications 8 à 15, et comprenant un
ou plusieurs véhicules et/ou diluants acceptables au plan pharmaceutique.

23. Composition pharmaceutique comprenant une molécule d'acide nucléique selon l'une quelconque des revendications 1 à 6, et comprenant un ou plusieurs véhicules et/ou diluants acceptables au plan pharmaceutique.

24. polypeptide recombinant selon l'une quelconque des revendications 8 à 15, destiné à un usage comme médicament.

25. Polypeptide recombinant selon l'une quelconque des revendications 8 à 15, destiné à traiter l'asthme, une allergie ou un état exacerbé par la production d'IgE.

26. Utilisation d'un polypeptide recombinant selon l'une quelconque des revendications 8 à 15 dans la préparation d'un médicament visant à traiter l'asthme, une allergie ou un état exacerbé par la production d'IgE.

27. Procédé de production d'un polypeptide recombinant selon l'une quelconque des revendications 8 à 15, ledit procédé comprenant la mise en culture de cellules contenant la construction génétique selon la revendication 7 sur une période de temps et dans des conditions suffisantes pour exprimer la molécule d'acides nucléiques, contenue dans ladite construction génétique, en vue de la production du polypeptide recombinant, et l'isolement dudit polypeptide recombinant.

28. Cellules animales isolées, comprenant la construction génétique selon la revendication 7 et exprimant le polypeptide recombinant tel que défini selon l'une quelconque des revendications 8 à 15 et produit par le procédé selon la revendication 27.

29. Protéine de fusion comprenant une première partie capable d'interagir avec l'IL-13, ladite première partie étant codée par une molécule d'acide nucléique telle que définie selon l'une quelconque des revendications 1 à 6, et une seconde partie dérivée d'un récepteur dé l'hémopoïétine, d'un récepteur à activité tyrosine kinase, d'un récepteur du TNF/NGF ou d'un récepteur couplé à une protéine G.

30. Protéine de fusion selon la revendication 29, dans laquelle la seconde partie comprend l'intégralité, ou une partie fonctionnelle, de la protéine de liaison à l'IL-13, une chaîne α du récepteur de l'IL-4, une chaîne γ du récepteur de l'IL-2
ou un récepteur d'une cytokine impliquée dans l'asthme ou dans une allergie.

31. Anticorps selon la revendication 16 ou 17, destiné à une utilisation comme médicament.

32. Anticorps selon la revendication 16 ou 17, dans lequel ledit anticorps inhibe l'interaction d'un polypeptide selon l'une quelconque des revendications 8 à 15 avec la chaîne α du récepteur de l'IL-4.
